(19) Europäisches Patentamt
European Patent Office
Office européen des brevets

(11) Publication number : **0 399 716 B1**

# (12) EUROPEAN PATENT SPECIFICATION

(45) Date of publication of patent specification : **26.01.94 Bulletin 94/04**

(51) Int. Cl.$^5$ : **A61K 31/57**, A61K 9/18, A61K 47/48

(21) Application number : **90305200.9**

(22) Date of filing : **15.05.90**

## (54) Hypoallergenic steroidal anesthetic/hypnotic compositions.

(30) Priority : **24.05.89 US 356705**

(43) Date of publication of application : **28.11.90 Bulletin 90/48**

(45) Publication of the grant of the patent : **26.01.94 Bulletin 94/04**

(84) Designated Contracting States : **AT BE CH DE DK ES FR GB GR IT LI LU NL SE**

(56) References cited :
**EP-A- 0 326 196**
**US-A- 4 596 795**
**STN INTERNATIONAL INFORMATION SERVICES DATA BASE: CHEMICAL ABSTRACTS, vol. 109, no. 14, abstract no. 116044r, Columbus, Ohio US;**
**STN INTERNATIONAL INFORMATION SERVICES DATA BASE: CHEMICAL ABSTRACTS, vol. 112, no. 14, abstract 125116e, Columbus, Ohio, US; M.E. BREWSTER et al.: "Development of a non-surfactant formulation for alfaxalone through the use of chemically-modified cyclodextrins",**

(73) Proprietor : **INNOVET, INC.**
**1655 Palm Beach Lakes Blvd. Suite 510**
**West Palm Beach, Florida 33401 (US)**
Proprietor : **THE UNIVERSITY OF FLORIDA**
**223 Grinter Hall**
**Gainesville, Florida 32611 (US)**
Proprietor : **PHARMOS CORPORATION**
**101 E. 52nd Street 36th Floor**
**New York, New York 10022 (US)**

(72) Inventor : **Houdeshell, Jesse W.**
**1260 Southwest 19th Avenue**
**Boca Raton, Florida 33486 (US)**
Inventor : **Bodor, Nicholas S.**
**6219 Southwest 93rd Avenue**
**Gainesville, Florida 32608 (US)**
Inventor : **Brewster, Marcus E. III**
**6705 Southwest 44th Avenue**
**Gainesville, Florida 32608 (US)**

(74) Representative : **Pendlebury, Anthony et al**
**PAGE, WHITE & FARRER 54 Doughty Street**
**London WC1N 2LS (GB)**

EP 0 399 716 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to means for use in methods of eliciting anesthesia and/or a hypnotic response in vertebrate animals without provoking a significant allergic response, comprising steroidal anesthetic/hypnotic compositions which contain complexes of steroidal anesthetics/hypnotics with selected cyclodextrin derivatives and which are free of surfactants.

Steroidal anesthetics were first brought to the forefront in the early 1940's by Hans Selye, who proposed that an oxygen molecule be present at opposite ends of a steroid for it to possess anesthetic properties [Selye, Proc. Soc. Exp. Biol. Med. 46, 116(1941)]. The most potent of the steroidal anesthetics at the time was considered to be pregnanedione, which was reported to be superior to barbiturates in the induction and maintenance of anesthesia by offering a greater margin of safety and facilitating recovery through rapid metabolism and elimination of the steroid from the blood and liver [Selye, Anesth. Analg. 21, 41(1942)]. This process did not rely on drug redistribution.

Subsequently, many other steroids were studied for their anesthetic and hypnotic activity; see, for example, the literature cited in Davis et al United States Patent No. 3,714,352. A quick-acting injectable anesthetic, 3α-hydroxypregnane-11,20-dione or predione, having the structural formula

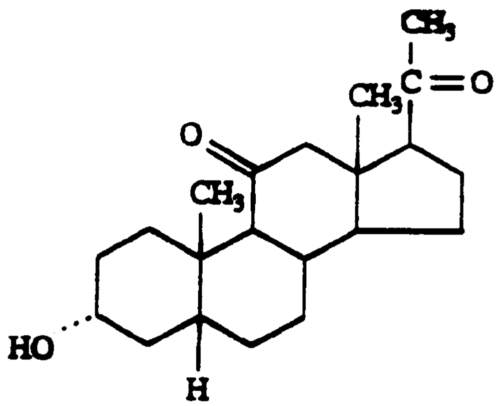

,

was described by Pitel et al in United States Patent No. 3,769,421. The corresponding 5α-epimer, 3α-hydroxy-5α-pregnane-11,20-dione, which has the structural formula

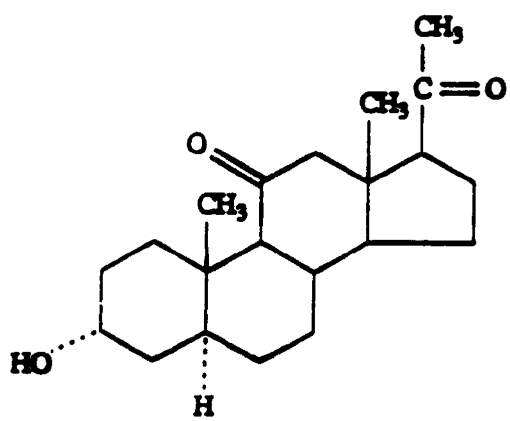

and which is also known as alfaxalone, was found to be a particularly promising steroidal anesthetic by Davis and coworkers at Glaxo Laboratories Limited. Both the 5α- and 5β-epimers had been previously described in the literature as having hypnotic and anesthetic properties, as acknowledged in Davis et al U.S. Patent No. 3,714,352, discussed below. After screening a number of anesthetic steroids, Davis et al reported that the presence of a free 3α-hydroxyl group was responsible for rapid induction and a high potency level of anesthesia, the most potent compound investigated being alfaxalone; Davis et al Steroid Anesthesia, Proceeding of the Conference of the Royal College of Physicians-London, 1972.

Because of the poor water solubility of alfaxalone, various attempts to enhance solubility were undertaken, including those described in Davis et al United States Patents No. 3,714,352; 3,781,435; 3,816,624; 3,917,830; 3,900,561; and 3,763,195.

Davis et al U.S. Patent No. 3,714,352 is directed to a method of inducing anesthesia in an individual (man or animal) by parenterally administering an aqueous solution containing at least 1 mg/mL of alfaxalone and an effective solubilizing amount of a parenterally acceptable non-ionic surface active agent. Examples of suitable non-ionic surface active agents, or surfactants, disclosed in the patent include Cremophor™ EL, a polyoxyethylated castor oil containing about 40 ethylene oxide units per triglyceride unit; Tween™ 80, polyoxyethylene sorbitan monooleate containing about 20 ethylene oxide units; Tween™ 60, polyoxyethylene sorbitan monostearate containing about 20 ethylene oxide units; and Tween™ 40, polyoxyethylene sorbitan monopalmitate containing about 20 ethylene oxide units.

Davis et al U.S. Patent No. 3,781,435 is directed to an anesthetic composition suitable for parenteral administratidn comprising alfaxalone in solution in an aqueous medium in an amount of at least 1 mg/mL of said medium, the medium also containing at least one further steroid of the formula

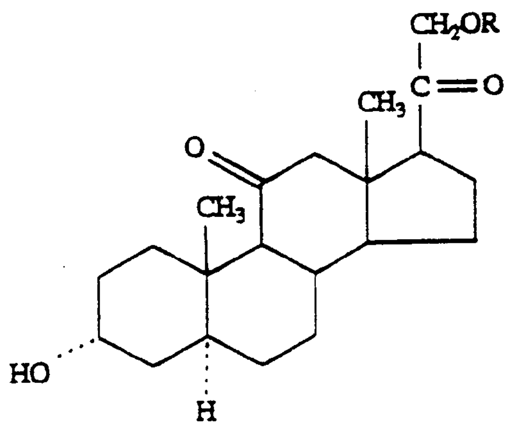

wherein R is lower alkanoyl, hemisuccinyl or benzoyl, for increasing the solubility of alfaxalone, the medium also containing at least 1% by weight of a parenterally acceptable non-ionic surface active agent having a HLB value of at least 9. When R in the foregoing formula is $COCH_3$, the steroidal solubility promoter is known as alfadolone acetate. Related parenteral formulations and methods of use are also described in this patent.

Steroids of the foregoing formula not only promote the solubility of alfaxalone, but themselves possess anesthetic activity (albeit of a generally lower level than alfaxalone). Solutions containing both alfaxalone and alfadolone acetate (or other compound of the foregoing formula) could therefore have a greater anesthetic potency than alfaxalone alone, due to the increased amount of alfaxalone dissolved and also the anesthetic activity of the additional steroid. See also Davis et al U.S. Patent No. 3,763,195.

Various other anesthetic compositions containing alfaxalone are described in Davis et al U.S. Patent No. 3,816,624, containing specific amounts or particle sizes of alfaxalone and specific amounts of surface active agent. Compositions in solution form contain at least 1 mg/mL of alfaxalone, while those in suspension form contain from 0.1 - 2% by weight of alfaxalone. Unit dosage forms for human use contain 10 - 300 mg of alfaxalone.

Davis et al U.S. Patent No. 3,917,830 describes a composition adapted for use in medicine by intravenous injection as an anesthetic comprising alfaxalone in solution in an inert liquid injection medium consisting essentially of a solution of water in an organic liquid selected from polyhydric alcohols having 2 - 3 OH groups and 1 - 6 carbon atoms and water-soluble esters of $\alpha$-hydroxycarboxylic acids with $C_{1-6}$ alkanols in which the organic liquid constitutes more than 50% of the total injection liquid, the composition being in unit dosage form. A method of inducing anesthesia using the described composition is also disclosed.

As yet another approach to the solubility problems, water-soluble salts of alfaxalone and other steroidal anesthetics have been utilized. Davis et al U.S. Patent No. 3,900,561 is directed to an aqueous solution for use as an i.v. anesthetic consisting essentially of from 0.1 - 20% by weight of a steroidal anesthetic having a solubility in water at 20°C of at least 1 mg/mL and from 0.1 - 50% by weight of a vein-damage reducing substance consisting of a parenterally acceptable non-ionic surface active component having an HLB value above 9 and not more than 30, whereby the physiological compatibility of the steroidal anesthetic is improved. Typical water-soluble salts include hydroxydione sodium succinate and the 3-hemisuccinate sodium salt of alfaxalone,

while Cremophor EL and Tween 80 are typical surface active components.

Yet other steroidal anesthetics/hypnotics have been developed subsequently to alfaxalone. Phillipps et al U.S. Patents No. 3,869,451 and 3,952,031 describe anesthetic steroids of the pregnane and 19-norpregnane series having a variety of substituents in the 2β-position (acyloxy, ether, thioether, alkyl, cycloalkyl, aryl, aralkyl, hydroxy, thiocyanato, nitro-oxy, halo, azido, sulfonyloxy or acylthio), a 3α-hydroxy group and a 5α-hydrogen and esters and 20-ketals thereof, with the 11-position having either two hydrogen atoms or an oxo group. Phillipps et al U.S. Patent No. 3,880,896 describes steroidal anesthetics of the pregnane and 19-norpregnane series, the compounds having a 5α-hydrogen atom or a 4,5-double bond, a 3α-hydroxy group, a 17α-hydrogen atom, a 20-oxo group and a 21-fluorine atom. Phillipps et al U.S. Patents No. 3,882,151 and 3,969,345 describes anesthetic 3α-oxygenated pregnane 21-ethers possessing a hydroxy group in the 3α-position; a hydrogen atom or a methyl group at the 10-position; a hydrogen atom in the 17α-position; a keto group in the 20-position; and an etherified hydroxyl group in the 21-position. Phillipps et al U.S. Patent No. 3,883,569 describes anesthetic steroids of the 5α-pregnane series possessing a 3α-hydroxy group, a 10-hydrogen atom or methyl group, an 11-oxo group, a 17α-hydrogen atom, a 20-oxo group and a group of the formula -XR at the 21-position, wherein -XR represents -OCOR, -O-COOR, -CCOSR or -OCONHR and R represents (a) a solubilizing group containing a basic nitrogen atom, (b) a nitrobenzyl group, (c) a lower alkyl group (when X is not -OCO-) or (d) a halogenoalkyl group. Cook et al U.S. Patent No. 3,953,429 describes anesthetic steroids of the androstane and pregnane series possessing a 2α-hydrogen or halogen or an alkyl group; a 3α-hydroxy or acyloxy group; a 3β-hydrogen or alkyl group; an 11β-hydrogen or hydroxy group or an epoxy group linked also to the 9-position; an 11α-hydrogen or alkyl or allyl group; or an 11-oxo group; a 16-hydrogen or halogen or a methyl or dimethyl group; a 20-oxo or ethylenedioxy group; and a 20-methyl or alkoxy group; which may be unsaturated at the 1(2),8(9) or 9(11) positions. Phillipps et al U.S. Patent No. 3,983,111 describes steroid anesthetics of the pregnane and 19-norpregnane series having a 3α-hydroxy group, a 17α-hydrogen atom, a 20-oxo group and at the 21-position a cyano, azido or basic azido group. Engelfried et al U.S. Patent No. 4,029,777 describes CNS-depressant (especially anesthetic-narcotic) 18-methyl-19-nor-20-ketopregnanes wherein the 3α and 3β substituents collectively are an oxygen atom, or each is hydrogen, or one is hydrogen and the other is hydroxy; the 11-substituent is a ketonic oxygen atom or two hydrogen atoms; and the hydrogen atom at the 5-position can be in the α- or β-configuration.

A few of the compounds described in the various patents summarized in the preceding paragraph may themselves be sufficiently water soluble for use intravenously; these would be acid addition salts of certain compounds, e.g. those containing a basic nitrogen atom. Most of the steroidal anesthetics described, however, are poorly soluble (insoluble or only sparingly soluble) in water and therefore are formulated for parenteral administration in an aqueous solution of a parenterally acceptable non-ionic surface active agent such as Cremophor EL. Indeed, the Phillipps et al and Cook et al patents suggest that these surface active agents may also be used even when the steroid is sufficiently water soluble, so as to reduce the risk of thrombophlebitis. Compare Davis et al U.S. Patent No. 3,900,561, discussed hereinabove.

Despite their solubility problems, the steroidal anesthetics have many desirable characteristics which make them attractive for human and veterinary use. Alfaxalone in particular has an excellent profile of rapid onset or induction, high therapeutic index and rapid offset effect. Child et al, Brit. J. Anesth. 43, 2(1971); Cambell et al, Brit. J. Anesth. 43, 14 (1971).

Alfaxalone was introduced to the market in 1971. In the marketed formulation, known as ALTHESIN™ (for human use) or SAFFAN™ (for veterinary use), alfaxalone was dissolved in the non-ionic detergent Cremophor EL and combined with an additional steroid, alfadolone acetate, to further enhance the solubility of alfaxalone in the Cremophor EL vehicle.

The ALTHESIN™/SAFFAN™ product was found to be very useful in that it was available in a stable solution, requiring little time to prepare for usage and it produced sleep in a single arm-brain circulation time after a single dose. In man, the ALTHESIN™ anesthetic was found to be comparable to other anesthetic agents in its cardiovascular effects and did not exhibit nearly the same degree of respiratory depression upon increased dosing. Recovery from the anesthetic was also deemed favorable. Corssen et al, Intravenous Anesthesia and Analgesia, Lea and Febiger, Philadelphia, p. 267-280(1988).

In contrast to inhalation anesthetics, intravenous anesthetics such as ALTHESIN™ are considered more attractive chemically and economically. Further, inhalation anesthetics pose a danger to operating room personnel because trace amounts of the anesthetic are present, which can cause a variety of ailments. Intravenous anesthetics are particularly well-suited for the induction of anesthesia and for short procedures, including many performed in small animals and other veterinary species. Unfortunately, however, use of the ALTHESIN™/SAFFAN™ formulations has been associated with the occurrence of an unacceptably high incidence of allergic reactions resembling anaphylactic shock; Tachon et al, Brit. J. Anesth. 55, 715-717 (1983). Indeed, the incidence of histaminoid-type reactions in humans has been estimated at from 1 in 11,000 to as high as 1

in 900-1,000: Clarke, Drugs 22, 26-41(1981) and references cited therein. These toxicity reactions occurred with a frequency sufficient to cause removal of the human formulation from the European market in the spring of 1984: Ennis et al, Agents and Actions 16, 265-268(1985). Neither the human nor the veterinary product was ever approved for use in the United States.

The allergic responses to ALTHESIN™ in man have varied from an erythematous rash and urticaria to bronchospasm, severe involuntary muscle movements, cardiovascular collapse and cardiac arrest: Corssen et al, Intravenous Anesthesia and Analgesia, Lea and Febiger, Philadelphia, p. 273(1988); Clarke, Drugs 22, 26-41(1981); Shipton, S. Afr. Med. J. 67, 1023-1025(1985); Watkins, British Journal of Hospital Medicine, 45-48 (July 1986). It is clear from studies in dogs that histamine release is involved in the use of the marketed alfaxalone/alfadolone acetate/Cremophor EL formulation, as characterized by severe cardiovascular collapse, with substantial and protracted decrease in blood pressure and cardiac output, and massive increase in plasma levels of histamine, as well as extensive flushing, other skin reactions and edema: Gaudy et al, Canad. J. Anaesth. 34, 122-129(1987); Ennis et al, Agents and Actions 20, 219-222(1987); Lorenz et al, Agents and Actions 12, 64-80 (1982). Indeed, the veterinary formulation is contraindicated in dogs because of histamine release problems and thus alfaxalone has been unavailable for safe use in this important segment of the pet population. Even in cats, for which the product was approved in a few countries outside the United States, less serious manifestations of histamine release (swollen ears, puffy paws and muzzles) are not uncommon. An antihistamine is sometimes administered to reduce this possibility: Lumb et al, Veterinary Anesthesia, second edition, Lea and Febiger, Philadelphia, pp. 312-315(1984).

Numerous studies have been undertaken to investigate the cause or causes of the allergic reactions observed in man, in dogs and in certain other animals following administration of ALTHESIN™ /SAFFAN™. Possible causes include either or both of the steroids present, the surfactant Cremophor EL and the steroid/Cremophor combination. It has been reported that while Cremophor EL alone does not cause an allergic reaction in man, it does cause such a reaction (as measured by histamine release) in dogs on the first exposure and in pigs on the second exposure. See Lorenz et al, Agents and Actions 12, 64-80(1982); Gaudy et al, Canad. J. Anaesth. 34, 122-129(1987); Ennis et al, Agents and Actions 20, 219-222(1987). While the evidence has been circumstantial, Cremophor EL has been accepted by many as the cause of the adverse allergic effects of the commercial formulation, e.g. Clarke, Drugs 22, 26-41(1981); Lumb et al, Veterinary Anesthesia, second edition, Lea and Febiger, Philadelphia, pp. 312-315(1984); Green, Animal Anaesthesia, Laboratory Animals Ltd., London, pp. 35-36(1979).

Nevertheless, it would appear that laying the blame for allergic reactions completely on the Cremophor vehicle oversimplifies the problem and does not adequately explain some of the scientific findings. This conclusion, for example, does not explain the reports that Cremophor alone does not cause histamine release in man; see, for example, Lorenz et al, Agents and Actions 7, 63-67(1977) and references cited therein; Ennis et al, Agents and Actions 16, 265-268(1985) and references cited therein. Indeed, a number of researchers have indicated that it is the combination of steroids with surfactant which causes ALTHESIN-related hypersensitivity, not the surfactant alone. As pointed out by Tachon et al, Br. J. Anaesth. 55, 715-717(1983), Cremophor's capacity to induce histamine release is unlikely to be a frequent mechanism for hypersensitivity, given the much lower frequency of allergic reaction to propanidid, another anesthetic drug formulated in Cremophor. Tachon et al and other researchers cited by them suggest complement involvement or an immune-mediated response as other possible mechanisms; see also Clarke et al, Drugs 22, 26-41(1981) and Watkins, British Journal of Hospital Medicine, 45-48 (July 1986). Ennis et al have reported that detergents such as Cremophor appear to potentiate the histamine release elicited by drugs. Those researchers, who have studied isolated mast cell systems, had earlier found histamine release with ALTHESIN™ but not with Cremophor EL in such systems: Ennis et al, Agents and Actions 18, 235-238(1986) and references cited therein. In clinical situations, the detergent may even potentiate the histamine release caused by yet other drugs given at about the same time.

Despite the fact that Cremophor EL has been implicated in the allergic reactions provoked by ALTHESIN™ administration, no definitive explanation of the nature and causes of the hypersensitivity has emerged. It is clear, however, that the steroidal anesthetics in general and alfaxalone in particular are valuable drugs which would be much more widely used if they could achieve their therapeutic purpose without provoking a significant allergic response.

Cyclodextrins are cyclic oligosaccharides. The most common cyclodextrins are a α-cyclodextrin, which is composed of a ring of six glucose residues; β-cyclodextrin, which is composed of a ring of seven glucose residues; and γ-cyclodextrin, which is composed of a ring of eight glucose units. The inside cavity of a cyclodextrin is lipophilic, while the outside of the cyclodextrin is hydrophilic; this combination of properties has led to widespread study of the natural cyclodextrins, particularly in connection with pharmaceuticals, and many inclusion complexes have been reported. β-Cyclodextrin has been of special interest because of its cavity size, but its relatively low aqueous solubility has limited its use in the pharmaceutical field.

Attempts to modify the properties of the natural cyclodextrins have resulted in the development of heptakis (2,6-di-O-methyl)-β-cyclodextrin, heptakis (2,3,6-trio-O-methyl)-β-cyclodextrin, hydroxypropyl-β-cyclodextrin, β-cyclodextrin-epichlorohydrin polymer and others. For a comprehensive review of cyclodextrins and their use in pharmaceutical research, see Pitha et al, in Controlled Drug Delivery, ed. S.D. Bruck, Vol. I, CRC Press, Boca Raton, Florida, pp. 125-148(1983). For an even more recent overview, see Uekama et al, in CRC Critical Reviews in Therapeutic Drug Carrier Systems, Vol. 3(1), 1-40 (1987); Uekama, in Topics in Pharmaceutical Sciences 1987, eds. D.D. Breimer and P. Speiser, Elsevier Science Publishers B.V. (Biomedical Division), 181-194(1987); and Pagington, Chemistry in Britain, pp. 455-458 (May 1987).

Inclusion complexes of α-,β- or γ-cyclodextrin or their mixtures with a variety of drugs have been described by numerous parties and various advantages have been attributed to the complexes. In the steroid area, reference may be made to Lipari United States Patent No. 4,383,992, which describes inclusion complexes of β-cyclodextrm itself with a variety of steroidal hormones (corticosteroids, androgens, anabolic steroids, estrogens and progestagens). The complexes are said to have improved water solubility and increased therapeutic response in the eye. However, as noted above, β-cyclodextrin has low aqueous solubilty (about 1.8% w/v at 25°C) with attendant nephrotoxicity.

Hydroxypropyl-β-cyclodextrin (HPCD) and its preparation by propylene oxide addition to β-cyclodextrin were described in Gramera et al United States Patent No. 3,459,731 nearly 20 years ago. Gramera et al also described the analogous preparation of hydroxyethyl-β-cyclodextrin by ethylene oxide reaction with β-cyclodextrin. Much more recently, Pitha and co-workers have described the improved preparation of this cyclodextrin derivative and its effects on the dissolution of various drug molecules. Pitha United States Patent No. 4,596,795, dated June 24, 1986, describes inclusion complexes of sex hormones, particularly testosterone, progesterone, and estradiol, with specific cyclodextrins, preferably hydroxypropyl-β-cyclodextrin and poly-β-cyclodextrin. The complexes enable the sex hormones to be successfully delivered to the systemic circulation via the sublingual or buccal route; the effectiveness of this delivery is believed to be due to "the high dissolution power of hydrophilic derivatives of cyclodextrins, the non-aggregated structure of their complexes with steroids, and their low toxicity and irritancy of mouth tissue". Success with other cyclodextrins, including poly-γ-cyclodetrrin and hydroxypropyl-γ-cyclodetrrin, have also been noted in the Pitha patent. See also Pitha et al, J. Pharm. Sci., Vol. 74, No. 9,987-990 (September 1985), concerning the same and related studies. Pitha et al also describe in the J. Pharm. Sci. article the storage stability of tablets containing a testosterone-hydroxypropyl-β-cyclodetrrin complex and the lack of toxicity of the cyclodextrin itself, as well as the importance of the amorphous nature of the cyclodextrin derivatives and their complexes with drugs in improving dissolution properties.

The improved, optimized preparation and purification of hydroxypropyl-β-cyclodextrin has been recently described by Pitha et al, International Journal of Pharmaceutics 29, 73-82(1986). In the same publication, the authors have described increased water solubility for 32 drugs in concentrated (40 to 50%) aqueous solutions of hydroxypropyl-β-cyclodextrin; improved solubilization of acetaminophen, apomorphine, butylated hydroxytoluene, chlorthalidone, cholecalciferol, dexamethasone, dicumarol, digoxin, diphenylhydantoin, estradiol, estriol, ethinylestradiol-3-methyl ether, ethisterone, furosemide, hydroflumethiazide, indomethacin, iproniazid phosphate, 17-methyltestosterone, nitroglycerin, norethindrone, ouabain, oxprenolol, progesterone, retinal, retinoic acid (all trans and salt forms), retinol, spironolactone, sulpiride, testosterone and theophylline was noted. The authors indicated this to be an extension of their earlier work with hydroxypropyl-β-cyclodextrin, which was previously found effective for oral administration of the sex hormones to humans. Their later work reported in Pitha et al, International Journal of Pharmaceutics 29,73-82 (1986), has also been very recently described in Pitha United States Patent No. 4,727,064, dated February 23, 1988. That patent claims a composition containing an amorphous complex of cyclodextrin and a drug, and a method of producing a stabilizing amorphous complex of a drug and a mixture of cyclodextrins comprising (1) dissolving an intrinsically amorphous mixture of cyclodextrin derivatives which are water soluble and capable of forming inclusion complexes with drugs in water; and (2) solubilizing lipophilic drugs into aqueous media to form a solution and form a solubilized drug/cyclodextrin complex.

Uekama et al, CRC Critical Reviews in Therapeutic Drug Carrier Systems, Vol. 3(1), pp. 1-40 (1987), have described the characteristics of various cyclodextrins, including hydroxypropyl-β-cyclodextrin The authors have presented data showing improved solubilization in water in the presence of 15 mg/mL of HPCD for the drugs carmofur, diazepam, digitoxin, digoxin, flurbiprofen, indomethacin, isosorbide dinitrate, phenytoin, prednisolone, progesterone and testosterone. In a discussion of the metabolism and toxicity of cyclodextrins, Uekama et al have indicated that cyclodextrins at sufficiently high concentrations cause hemolysis, and that the methylated cyclodextrins have higher hemolytic activity than the natural cyclodextrins. Hydroxypropyl-β-cyclodextrin is said to cause hemolysis beginning at 4.5 mM. The authors have further indicated that parenteral administration of large doses of cyclodextrins should be avoided, but that "γ-cyclodextrin and hydroxypropyl-β-cyclodextrin seem to be useful in drug solubilization for injections and liquid preparations used for mucous membranes."

JANSSEN PHARMACEUTICA N.V.'s International Patent Application No. PCT/EP84/00417, published under International Publication No. WO85/02767 on July 4, 1985, has described pharmaceutical compositions comprising inclusion compounds of drugs, which are unstable or only sparingly soluble in water, with partially etherified β-cyclodextrin derivatives having hydroxyalkyl and optionally additional alkyl groups. Among the cyclodextrin derivatives contemplated is hydroxypropyl-β-cyclodextrin, while the drugs include non-steroidal antirheumatic agents, steroids, cardiac glycosides and derivatives of benzodiazepine, benzimidazole, piperidine, piperazine, imidazole and triazole. Preferred drugs include etomidate, ketoconazole, tubulazole, itraconazole, levocabastine and flunarizine. The pharmaceutical compositions of the invention include oral, parenteral and topical formulations, with 4 to 10% solutions of cyclodextrin derivatives being utilized to solubilize various drugs. Improved solubilities of indomethacin, digitoxin, progesterone, dexamethasone, hydrocortisone and diazepam using 10% HPCD are shown, and an injectable formulation of diazepam in 7% HPCD is specifically described. The relatively low cyclodextrin concentrations used reflect a desire to avoid or minimize the hemolytic effects observed at higher cyclodextrin concentrations.

Carpenter et al, The Journal of Pediatrics, 111, 507-512 (October 1987) describe intravenous infusion of 2-hydroxypropyl-β-cyclodextrin, prepared as a 5% solution in water, to treat severe hypervitaminosis A. It was found that, during infusion, circulating retinyl esters increased transiently, while total vitamin A excreted in the urine was enhanced after infusion. Thus, intravenous infusion of 5% HPCD was found to decrease in vivo levels of the vitamin, presumably by complexing with the vitamin and removing some of the excess from the body.

JANSSEN PHARMACEUTICA N.V.'s European Patent Application No. 86200334.0, published under EPO Publication No. 0197571 on October 15, 1986, describes γ-cyclodextrin derivatives which are cyclodextrin substituted with $C_1$-$C_8$ alkyl, hydroxy $C_1$-$C_8$ alkyl, carboxy $C_1$-$C_8$ alkyl or $C_1$-$C_8$, alkyloxycarbonyl $C_1$-$C_8$ alkyl or mixed ethers thereof. Among the specific derivatives named are hydroxypropyl-γ-cyclodextrin and hydroxyethyl-γ-cyclodextrin. Compositions comprising the cyclodextrin derivatives and a drug are also described. See also corresponding Müller United States Patent No. 4,764,604, dated August 16, 1988.

The inclusion characteristics of yet other derivatized cyclodextrins have also been described in the literature. Studies of branched cyclodextrins which are glucosyl and maltosyl derivatives of α-,β- and γ-cyclodextrin and their inclusion complexes with drugs have recently been reported. Uekama, in Topics in Pharmaceutical Sciences 1987, eds. D. D. Breimer and P. Speiser, Elsevier Science Publishers B. V. (Biomedical Division), 181-194(1987), has described the effects on bio-pharmaceutical properties of maltosyl and glucosyl cyclodextrin derivatives, including enhanced drug absorption.

Koizumi et al, Chem. Pharm. Bull. 35 (8), 3413-3418(1987), have reported on inclusion complexes of poorly water-soluble drugs with glucosyl cyclodextrins, namely 6-O-α-D-glucosyl-α-CD ($G_1$-α-CD), 6-O-α-D-glucosyl-β-CD ($G_1$-β-CD) and 6A, $6^D$-di-O-α-D-glucosyl-β-CD ($2G_1$-β-CD).

Okada et al, Chem. Pharm. Bull., 36 (6), 2176-2185(1988), have reported on the inclusion complexes of poorly water-soluble drugs with maltosyl cyclodextrins, namely 6-O-α-maltosyl-α-CD ($G_2$-α-CD), 6-O-α-maltosyl-β-CD ($G_2$-β-CD), 6-O-α-maltosyl-γ-CD ($G_2$-γ-CD), 6-O-α-maltotriosyl-α-CD ($G_3$-α-CD), 6-O-α-maltotriosyl-β-CD ($G_3$-β-CD) and 6-O-α-maltotriosyl-γ-CD ($G_3$-γ-CD).

Yamamoto et al, in International Journal of Pharmaceutics 49, 163-171(1989), have described physicochemical properties of branched β-cyclodextrins such as glucosyl-β-cyclodextrin, maltosyl-β-cyclodextrin and dimaltosyl-β-cyclodextrin, and their inclusion characteristics. Those authors report that the branched β-cyclodextrins are better solubilizers for poorly water-soluble drugs and have less hemolytic activity than β-cyclodextrin itself, and they suggest that glucosyl-β-cyclodextrin and maltosyl-β-cyclodextrin may be especially useful in parenteral preparations.

The patent literature reflects much recent work on the branched cyclodextrins carried out by Japanese workers, as discussed below.

Japanese Kokai 63-135402 (TOKUYAMA SODA KK), published June 7, 1988, describes compositions consisting of maltosyl-β-cyclodextrin and at least one of digitoxin, nifedipine, flulubiprophene, isosorbide nitrate, phenytoin, progesterone or testosterone. The compositions have improved water solubility and reduced erythrocyte destruction, are safe for humans and can be used as injections, eye drops, syrups, and for topical and mucous membrane application.

Japanese Kokai 62-281855 (DAIKIN KOGYO KK), published December 7,1987, describes stable, water-soluble inclusion compounds of maltosyl-β-cyclodextrin with a variety of vitamins and hormones, e.g. steroid hormones such as prednisolone, hydrocortisone and estriol. These lipophilic vitamins and hormones can thus be used as aqueous solutions.

Japanese Kokai 63-036793 (NIKKEN CHEM KK), published February 17,1988, describes the preparation of dimaltosyl-γ-cyclodextrin and its general use in medicines.

Japanese Kokai 62-106901 (NIKKEN CHEM KK), published May 18, 1987, describes the preparation of digiucosyl-β-cyclodextrin and its general use for pharmaceuticals.

Japanese Kokai 61-236802 (NIKKEN CHEM KK), published October 22, 1986, describes the preparation of maltosyl-γ-cyclodextrin and its general use with drugs.

Japanese Kokai 61-197602(NIKKEN CHEM KK), published September 1, 1986, describes the preparation of maltosyl-β-cyclodextrin and its expected use in medicines.

Japanese Kokai 61-070996 (NIKKEN CHEM KK), published April 11, 1986, describes the preparation of maltosyl-α-cyclodextrin and its general use in pharmaceuticals.

Japanese Kokai 63-027440 (SANRAKU OCEAN), published February 5, 1988, describes compositions containing a water-insoluble or slightly soluble drug together with glucosylated branched cyclodextrin. Among the drugs mentioned are steroid hormones.

Japanese Kokai 62-164701 (SHOKUHIN SANGYO BIO), published July 21, 1987, describes the preparation of diglucosyl-α-cyclodextrin and its general use in medicine.

Japanese Kokai 62-003795 (TOKUYAMA SODA KK), published January 9, 1987, describes production of glucose and maltoligosaccharide (2-4 glucose units) derivatives of α-, β- and γ-cyclodextrin and their use as stabilizers for pharmaceuticals.

The present invention provides means for use in a method for eliciting anesthesia or a hypnotic response in a vertebrate animal without provoking a significant allergic response.

The present invention further provides hypoallergenic steroidal anesthetic/hypnotic compositions and methods for their preparation.

Still further, the present invention provides methods of use for the steroidal anesthetic/hypnotic alfaxalone and formulations containing same.

Thus, in one embodiment of the present invention the compositions are adapted to a method for eliciting anesthesia in a vertebrate animal without provoking a significant allergic response, said method comprising parenterally administering to said animal a parenterally acceptable hypoallergenic composition comprising an anesthetically effective amount of a steroidal anesthetic complexed with cyclodextrin selected from the group consisting of hydroxypropyl, hydroxyethyl, glucosyl, maltosyl and maltotriosyl derivatives of β- and γ-cyclodextrin, the amount of said steroidal anesthetic being from about 0.5% to about 15% by weight of the amount of said cyclodextrin in said composition, said composition being substantially free of surface active agents.

In another embodiment, the present invention provides a method for preparing a parenterally acceptable hypoallergenic steroidal anesthetic composition, said method comprising complexing an anesthetically effective amount of a steroidal anesthetic with cyclodextrin selected from the group consisting of hydroxypropyl, hydroxyethyl, glucosyl, maltosyl and maltotriosyl derivatives of β- and γ-cyclodextrin, followed by formulating the resultant complex into a parenterally acceptable composition wherein the amount of said steroidal anesthetic is from about 0.5% to about 15% by weight of the amount of said cyclodextrin in said composition, said composition being substantially free of surface active agents.

In still another embodiment, the present invention provides a method for preparing a parenterally acceptable hypoallergenic steroidal hypnotic composition, said method comprising complexing a hypnotically effective amount of a steroidal hypnotic with cyclodextrin selected from the group consisting of hydroxypropyl, hydroxyethyl, glucosyl, maltosyl and maltotriosyl derivatives of β- and γ-cyclodextrin, followed by formulating the resultant complex into a parenterally acceptable composition wherein the amount of said steroidal hypnotic is from about 0.5% to about 15% by weight of the amount of said cyclodextrin in said composition, said composition being substantially free of surface active agents.

In yet another embodiment, the present invention provides a parenterally acceptable hypoallergenic steroidal anesthetic composition for use in eliciting anesthesia in a vertebrate animal without provoking a significant allergic response, which composition comprises an anesthetically effective amount of a steroidal anesthetic complexed with cyclodextrin selected from the group consisting of hydroxypropyl, hydroxyethyl, glucosyl, maltosyl and maltotriosyl derivatives of β- and γ-cyclodextrin, the amount of said steroidal anesthetic being from 0.5% to 15% by weight of the amount of said cyclodextrin, said composition being substantially free of surface active agents.

In still another embodiment, the present invention provides a parenterally acceptable hypoallergenic steroidal hypnotic composition for use in eliciting a hypnotic response in a vertebrate animal without provoking a significant allergic response, which composition comprises a hypnotically effective amount of a steroidal hypnotic complexed with cyclodextrin selected from the group consisting of hydroxypropyl, hydroxyethyl, glucosyl, maltosyl and maltotriosyl derivatives of β- and γ-cyclodextrin, the amount of said steroidal hypnotic being from 0.5% to 15% by weight of the amount of said cyclodextrin, said composition being substantially free of surface active agents.

In preferred embodiments of the invention, the steroidal anesthetic/hypnotic is alfaxalone.

Other embodiments and advantages of the present invention will be apparent from the following detailed description and accompanying drawing, in which the sole **FIGURE** depicts the phase-solubility of alfaxalone in 2-hydroxypropyl-β-cyclodextrin (HPCD) in buffered (●) and in unbuffered (○) media. Molar concentrations of drug solubilized are plotted against increasing molar concentrations of HPCD. The information in the boxed area refers to the unbuffered (○) system.

The expression "parenteral" as used herein refers to routes of administration other than through the gastrointestinal tract or lungs, and to formulations for use in administering drugs by such routes. Thus, "parenteral" as used herein includes, for example, intravenous, intraarterial, intramuscular, subcutaneous, intra-articular (i.e. into the joint, which in turn includes intra-synovial, i.e. into the syniovial fluid), intracranial, intracerebral-ventricular, intrathecal and intraperitoneal, especially intravenous, intramuscular and subcutaneous routes and formulations. Although presently preferred parenteral routes and formulations are injectable, especially when anesthesia is desired, the term "parenteral" is used in its broadest sense herein and thus also includes such routes and formulations as buccal, sublingual, gingival, nasal, rectal, vaginal and intraocular.

The expression "vertebrate animal" as used herein encompasses all animals which are characterized by a segmented bony or cartilaginous spinal column, including fishes, amphibians, reptiles, birds and mammals. Both domesticated and wild animals are intended, as well as humans and semi-domesticated animals. Domesticated animals of particular interest include companion animals such as dogs and cats, and those of economic interest such as horses, swine, sheep, goats, llamas, rabbits, cattle, donkeys, mules and poultry. Semi-domesticated animals include caged pet animals such as birds and small rodents, as well as rabbits and ferrets. Zoo animals of particular interest include exotic birds and wild beasts such as bison, buffalo, elk, wild cats, monkeys, apes, chimpanzees, kangaroos, alligators and snakes. Obviously, most animal families have both domesticated or semi-domesticated and wild members, and those named here are merely representative.

Vertebrate animals of especially great interest herein are those which are particularly prone to allergic reaction to histamine-inducers in general and to the ALTHESIN™/SAFFAN™ formulation in particular. These include humans, dogs, horses, cattle, swine and, to a lesser extent, cats.

The term "hypoallergenic composition" as used herein is intended to mean a composition having reduced or below-normal tendency to act as an allergen, an allergen being an antigenic substance capable of producing immediate-type hypersensitivity. The compositions provided by the present invention have substantially reduced allergic potential as compared to the commercial surfactant-containing alfaxalone formulations.

The expression "allergic response" is used herein to encompass the various manifestations of a hypersensitivity or allergic reaction to parenteral anesthetics, whether caused by anaphylactic (immune) or anaphylactoid (non-immune) reactions. These manifestations include various combinations of flushing, urticaria, hypotension and bronchospasm. See Watkins, British Journal of Hospital Medicine, pp. 45-48 (July 1986). Although other mediators may play a role, histamine is present at varying levels in all anaphylactoid reactions and thus high histamine plasma levels are indicative of an allergic response. See also the discussion of various allergic manifestations in humans and dogs discussed hereinabove with respect to the ALTHESIN™/SAFFAN™ formulations.

Steroidal anesthetics/hypnotics for use in the methods and compositions of the present invention are those which are "lipophilic" or "lipid-soluble", i.e. those which are insoluble or sparingly soluble or unstable in water. These include predione (described, for example, in Pitel et al U.S. Patent No. 3,769,421), alfaxalone (described, for example, in Davis et al U.S. Patents No. 3,714,352 and 3,816,624), alfadolone acetate and congeners (described, for example, in Davis et al U.S. Patents No. 3,781,435 and 3,763,195) and any of the other relatively water-insoluble steroidal anesthetics/hypnotics described in the art, for example, those described in Phillipps et al U.S. Patents No. 3,869,451; 3,952,031; 3,880,896; 3,882,151; 3,969,345; 3,883,569; and 3,983,111; Cook et al U.S. Patent No. 3,953,429; and Engelfried et al U.S. Patent No. 4,029,777; as well as various combinations of the steroids described in these patents, e.g. the combination of alfaxalone and alfadolone acetate.

The preferred steroidal anesthetic/hypnotic for use in the present invention is alfaxalone. That steroid is extremely lipophilic; its water solubility is less than 5 μg/mL

The cyclodextrins contemplated for use herein are hydroxypropyl, hydroxyethyl, glucosyl, maltosyl and maltotriosyl derivatives of β-cyclodextrin and the corresponding derivatives of γ-cyclodextrin. The hydroxyalkyl groupings may contain one or more hydroxyl groups, e.g. hydroxypropyl (2-hydroxypropyl, 3-hydroxypropyl), dihydroxypropyl and the like. The glucosyl, maltosyl and maltotriosyl derivatives may contain one or more sugar residues, e.g. glucosyl or diglucosyl, maltosyl or dimaltosyl. Various mixtures of the cyclodextrin derivatives may be used as well, e.g. a mixture of maltosyl and dimaltosyl derivatives. Specific cyclodextrin derivatives for use herein include hydroxypropyl-β-cyclodextrin (HPCD or HPBCD), hydroxyethyl-β-cyclodextrin (HEBCD), hydroxypropyl-γ-cyclodextrin (HPGCD), hydroxyethyl-γ-cyclodextrin (HEGCD), dihydroxypropyl-β-cyclodextrin (2HPBCD), glucosyl-β-cyclodextrin ($G_1$-β-CD or $G_1$BCD), digiucosyl-β-cyclodextrin ($2G_1$-β-CD or $2G_1$-β-BCD), maltosyl-β-cyclodextrin ($G_2$-β-CD or $G_2$BCD), maltosyl-γ-cyclodextrin ($G_2$-γ-CD or $G_2$GCD), maltotriosyl-

β-cyclodextrin ($G_3$-β-CD or $G_3$BCD), maltotriosyl-γ-cyclodextrin ($G_3$-γ-CD or $G_3$GCD) and dimaltosyl-β-cyclodextrin ($2G_2$-β-CD or $2G_2$BCD), and mixtures thereof such as maltosyl-β-cyclodextrin/dimaltosyl-β-cyclodextrin.

Hydroxypropyl-β-cyclodextrin for use in the methods of the present invention is commercially available. Alternatively, it may be prepared by known methods, especially by use of the optimized procedure of Pitha et al, International Journal of Pharmaceutics, 29, 73-82(1986). The following is a typical procedure using the Pitha et al method:

31 g of sodium hydroxide were dissolved in 250 mL of water. Then, 100 g of β-cyclodextrin were added and the solvent was warmed to effect solution. The flask was cooled and 50 mL of propylene oxide were added. The flask was fitted with a dry ice/acetone condenser during the addition. The solution was allowed to come to room temperature and was stirred for 72 hours. The solution was then neutralized with concentrated hydrochloric acid and diluted with water. The solvent was removed in vacuo, leaving a syrup which was taken up in ethanol. Alter stirring for 30 minutes at room temperature, the sodium chloride produced was removed by filtration. The filter cake was washed with ethanol and the combined ethanol layers were reduced in vacuo. The residue was dissolved in water and dialyzed in cellulose acetate (#7,38 mm, 4.6 mL/cm, molecular weight cut off = 1000, Fisher Scientific). Alter 5 hours at 0° C, the solution was removed from the dialysis tubing and freeze-dried. The resulting solid was suspended in acetone and stirred overnight. The filtered solid was resuspended in acetone and stirred for 24 hours. The solid was collected by filtration and dissolved in 200 mL of water and then lyophilized. 75 grams of purified hydroxypropyl-β-cyclodextrin were obtained. The degree of substitution was calculated by NMR and by comparison with an authentic sample.

The Pitha et al method for preparation of HPCD by condensation of propylene oxide with β-cyclodextrin in alkaline aqueous solution unfortunately suffers from disadvantages, particularly in purification of the product. After completion of the condensation, the reaction mixture is neutralized with hydrochloric acid, water is evaporated under vacuum and the syrupy residue is dissolved in ethanol to precipitate sodium chloride, the main by-product of the reaction. After filtration, ethanol is evaporated under vacuum and the residue is dissolved in water and dialyzed to remove the remaining sodium chloride and polymerization products of propylene oxide. During dialysis, part of the hydroxypropyl-β-cyclodextrin goes through the membrane and is lost. The dialysate is then freeze-dried, twice stirred in acetone and washed to remove the remaining polymerization products. Finally, hydroxypropyl-β-cyclodextrin is freeze-dried again. The second freeze-drying is necessary because the product after washing with acetone is not homogeneous.

To overcome these difficulties with the Pitha et al process, a new method has been developed by Maciej Smulkowski of the University of Florida, Gainesville, Florida for the synthesis of HPCD. This new method involves removal of sodium hydroxide from the reaction mixture by an ion exchange resin ($H^+$); as a result, several time-consuming steps of Pitha et al's purification can be avoided. Moreover, the amount of sodium hydroxide used by Pitha et al (7 equivalents for one of β-cyclodextrin) can be decreased to 2 equivalents of sodium hydroxide per cyclodextrin molecule, and still produce a product with the appropriate NMR and optical rotation.

According to the new method, β-cyclodextrin is first condensed with propylene oxide in alkaline solution, sodium hydroxide is removed on an ion exchange column (Dowex™ 50W-X8, $H^+$ form), the eluate is evaporated under vacuum to one-half of the original volume, the remaining solution is freeze-dried, the resulting white solid is washed with acetone and freeze-dried again, then subjected to grinding and sieving. Possible modifications of this method include: (1) use of the ionic exchange resin for neutralization in the reaction flask, with filtration of the resin and washing on the filter funnel; (2) use of calcium, magnesium, lithium or potassim hydroxide to dissolve the cyclodextrin; (3) removal of hydroxides after the reaction by saturating the reaction mixture with carbon dioxide or neutralization with sulfuric acid in place of the ion exchange resin; (4) use of even less sodium hydroxide (between 1 and 2 equivalents); and (5) elimination of the second freeze-drying.

The following is a typical procedure using the new, improved method:

50 g of β-cyclodextrin was dissolved in a solution of 3.53 g of sodium hydroxide in 75 mL of water and treated with 29 mL of propylene oxide at 0°C. The reaction mixture was maintained for 5 hours at that temperature, then was kept at room temperature for 42 hours. At the end of that time, the reaction mixture was passed through the Dowex™ 50W-X8 column ($H^+$ form), the column was washed with water and the eluate was evaporated under vacuum to a volume of 100 mL, then freeze-dried. The resulting white solid was washed with acetone to give 51 g of HPCD, with the same degree of substitution (4.7) and NMR as the HPCD prepared by the Pitha et al method. Residue on ignition was 0.0%. Optical rotation also was identical to that of the Pitha et al product.

Condensation of 25 g of β-cydodextrin using 7.71 g of sodium hydroxide gave similar results.

A further improvement in the new, improved HPCD synthesis utilizes activated carbon for purification of the solution prior to the last freeze-drying. Thus, when the aqueous solution from the Dowex 50 ionic exchange column was treated with activated carbon, most of the polymerization products were removed without loss of

HPCD, and the filtrate after only one washing with ethyl acetate was ready for final freeze-drying. In this way, only one freeze-drying was required. Crystallization of the final product instead of freeze-drying is also possible, at least on a small scale.

The product from the modified new process (using activated carbon) appears to be superior to that of the original new process and the Pitha et al process. First, the product is snow white and produces a colorless aqueous solution, whereas solutions of the earlier products were yellow. Secondly, the product is not oily, which may be due to removal of more highly substituted, less soluble, oily cyclodextrins.

HPCD can be prepared in varying degrees of substitution, such as 5 or 7. Typically, the foregoing procedure is used to produce HPCD (ASDS 7). The mass spectra for the isomeric mixture of HPCD centers around 7 degrees of substitution. This spectra is obtained by "softly" ionizing the sample using fast atom bombardment. The generated spectra is similar to those previously reported (obtained by Californium-252 plasma desorption) in both the symmetry of the isomeric distribution and the numerical spread of the isomers formed.

Hydroxyethyl-β-cyclodextrin (HEBCD) can be prepared analogously to HPCD, utilizing the improved procedure detailed above but substituting an equivalent quantity of ethylene oxide for the propylene oxide there employed.

The synthesis of 2-hydroxypropyl-γ-cyclodextrin (HPGCD) similarly uses the same basic procedure as for HPCD, substituting γ-cyclodextrin for the β-cyclodextrin starting material. However, because γ-cyclodextrin contains eight glucose residues compared to seven for β-cyclodextrin, the amount of propylene oxide used can be reduced in order to lower the degree of substitution. Use of 0.75 mole of propylene oxide per 0.077 mole of γ-cyclodextrin (~20% excess considering 8 OH groups) affords HPGCD with a degree of substitution of 8, while use of 0.56 mole of propylene oxide (~10% less than equivalent) gives a degree of substitution of about 7.

Hydroxyethyl-γ-cyclodextrin (HEGCD) can be prepared similarly to HPGCD as described in the preceding paragraph, simply using an equivalent quantity of ethylene oxide in place of the propylene oxide.

Thus, the hydroxyalkyl cyclodextrins intended for use herein can be prepared by procedures described by Pitha et al or variations thereof. Obtained by these procedures, the cyclodextrins are intrinsically amorphous mixtures. The importance of the amorphous nature of the cyclodextrins is described by Pitha et al, J. Pharm. Sci., Vol. 74, No. 9,987-990 (September 1985). The advantages of the amorphous nature of these materials are more pronounced at higher concentrations of cyclodextrin.

The other cyclodextrins intended for use in the present invention, i.e. the glucosyl, maltosyl and maltotriosyl derivatives of β- and γ-cyclodextrin, are branched cyclodextrins which are highly soluble in water compared to the parent cyclodextrins. These branched cyclodextrins can be produced by microbiological processes from the parent cyclodextrins. Glucosyl-β-cyclodextrins can be obtained from the mother liquor of a large-scale β-cyclodextrin synthesis with Bacillus ohbensis cyclomaltodextrin glucanotransferase; see Koizumi et al, Chem. Pharm. Bull., 35 (8), 3413-3418 (1987) and reference cited therein. Maltosyl and maltotriosyl β- and γ-cyclodextrins can be prepared from the parent cyclodextrin and maltose or maltotriose through the reverse action of Pseudomonas isoamylase or Klebsiella aerogenes pullulanase, while glucosyl-γ-cyclodextrin can be prepared by enzymatic hydrolysis of maltosyl-γ-cyclodextrin; see Okada et al, Chem. Pharm. Bull., 36 (6), 2176-2185(1988) and references cited therein. The preparation of maltosyl-β-cyclodextrin by reacting maltose with β-cyclodextrin in the presence of pullulanase is also described in Japanese Kokai 61-287902, published December 18, 1986, and Japanese Kokai 61-197602, published September 1, 1986. A mixture of maltosyl-β-cyclodextrin and various dimaltosyl-β-cyclodextrins may be conveniently employed. See also Kainuma et al United States Patent No. 4,668,626, issued May 26, 1987.

## EXPERIMENTAL SECTION

### SOLUBILITY AND STABILITY STUDIES

Micronized alfaxalone, 2-hydroxypropyl-β-cyclodextrin (HPCD) and 2-hydroxypropyl-γ-cyclodextrin (HPGCD) were used in the following studies. HPCD and HPGCD were synthesized according to procedures described hereinabove. The average degree of substitution for HPCD and HPGCD was found to be 7.1 and 7.6, respectively, when analyzed by fast atom bombardment (FAB) mass spectrometry using a Klatos MS80RFA double-focusing instrument fitted with a fast atom gun.

All cyclodextrin solutions were made using deionized water (Barnstead Nanopure II Ultrapure Water System). An excess of alfaxalone was sonicated in the appropriate 20% w/v cyclodextrin solution (HPCD or HPGCD) for 24 hours. The suspensions were then centrifuged and filtered through 0.45 μm polyvinylidine difluoride membranes (Millex, HV4, Millipore) and analyzed by HPLC. Phase solubility studies used concentrations of 1, 2, 5, 10, 15, 20, 30, 40 and 50% (w/v) HPCD. These solutions were prepared with or without a pH

7.4 isotonic buffer (phosphate). In preparing the freeze-dried complex, alfaxalone was sonicated in a 43.5% w/v solution of HPCD for 24 hours at which time the suspension was filtered. The filtrate was frozen in liquid nitrogen and lyophilized (Labconco Freeze Dried Model 18). The solid was then sieved (60 mesh) and the degree of drug incorporation determined by HPLC.

Alfaxalone in aqueous solutions was quantitated using HPLC. The system configuration included a Spectra Physics model 8810 reciprocating piston high pressure pump, an LDC/Milton Roy Spectromonitor D variable wavelength detection set at 208 nm, a Spectra Physics Model 4290 computing integrator and a Spectra Physics Model 8780 autosampler. Separation was effected on a 5μ Spherisorb C-18 ODS-2 reversed phase column (4.6 mm i.d. x 250 mm, Alltech Associates). The mobile phase consisted of methanol:$H_2O$ (65:35) and all determinations were conducted at ambient temperature. In this system, alfaxalone eluted at 15.5 min and butyl Paraben™, the internal standard, at 8.5 minutes. Standard curves for alfaxalone (response alfaxalone/response internal standard) were linear (r = 0.999) over the examined concentration range.

To determine the stability of alfaxalone/HPCD to steam sterilization, samples of alfaxalone (20 mg/mL) were prepared in 24.1% (w/v) HPCD which was either buffered (39 mM phosphate buffer, pH 7.4) or unmanipulated and placed in glass ampoules. The ampoules were sealed and segregated into three groups. One group was stored at 4°C, one at room temperature and one was autoclaved at 123°C (17 psi) for 35 minutes. The resulting solutions were then analyzed by HPLC. To ensure that these conditions gave an appropriate microbial kill, samples of the above cyclodextrin solutions were inoculated with Staphylococcus epidermidus and Escherichia coli (Department of Histopathology, VA Medical Center, Gainesville, FL) at concentrations of $10^7$ organism/mL. Samples were steam sterilized using the above conditions. The vials were opened in a sterile laminar flow hood and 300-400 μL of the solution placed on separate blood agar plates. The plates were incubated overnight and examined for bacterial growth.

The results of the screening of the solubility of alfaxalone in 20% aqueous solutions (w/v) of HPCD and HPGCD are shown in **TABLE I** below.

## TABLE I

Effect of Representative Cyclodextrins on Alfaxalone Solubility

| Cyclodextrin | Solubility | Increase in Aqueous Solubility[a] |
|---|---|---|
| HPCD | 29 mg/mL | 8056 |
| HPGCD | 19 mg/mL | 5278 |

[a] Increase in aqueous solubility relative to alfaxalone in unmodified aqueous solutions (3.6 μg/mL).

Thus, these cyclodextrins dramatically increased the aqueous solubility of alfaxalone. This represents an increase in the aqueous solubility of the steroid of approximately four orders of magnitude and is substantially more than the potency of the withdrawn commercial product, ALTHESIN™ (9.0 mg/mL).

For further study, HPCD was chosen. HPCD is highly water soluble and relatively hydrophilic. Toxicity studies in monkeys and rats have confirmed that HPCD is innocuous at relatively high doses when administered intravenously. Thus, in acute (14 day) and subchronic (90 day) paradigms in both cynomolgus monkeys and Sprague Dawley rats, no material-related changes were observed after i.v. dosing of 200 mg/kg to the animals every second day.

The **FIGURE** shows the phase-solubility profile for alfaxalone in HPCD with and without isotonic pH 7.4 phosphate buffer. As indicated, there is no significant effect of the buffer on complexation. The data indicate that an $A_L$-type relationship as described by Higuchi and Connor is produced [Higuchi et al, Adv. Anal. Chem. Instr. 4, 117 (1965)]. The slope of the phase solubility curve (in the absence of buffer) gives a value of 0.705 indicating a highly efficient cyclodextrin-steroid interaction. In addition, the $K_{1:1}$ calculated for this interaction based on the equation:

$$K_{1:1} = \frac{\text{slope}}{S_0\,(1 - \text{slope})}$$

was found to be 326078 $M^{-1}$ indicating a highly stable complex is formed. This value is approximately 10 times higher than those reported for other steroids. In this study, a 50% w/v solution of HPCD solubilized 76.9 mg of alfaxalone per mL of solution. This is almost 10-fold greater than the maximum solubility achieved in Cremophor™ using alfadolone acetate as a solubilizer.

Solutions of alfaxalone and HPCD were conveniently lyophilized to generate a white powder which was stable and easily reconstitutable. Upon reconstitution, the alfaxalone did not precipitate and the solution could be diluted so long as the cyclodextrin component was maintained at levels above 20% w/v. When a 43.5% w/v solution of HPCD was used in the complexation process, the lyophilized solid contained 126 mg of active drug per gram of complex.

A preliminary stability study was subsequently conducted to evaluate the potential for steam sterilization of aqueous formulations of alfaxalone. Alfaxalone at a concentration of 20 mg/mL was prepared in purified water containing 24.1% w/v HPCD with or without 39 mM (pH 7.4) phosphate buffer. The solutions were sealed in glass ampoules and were protected from light. Samples are then stored at room temperature or at 4°C. A third sample set was autoclaved (123°C, 35 min, 17 psi). As shown in **Table II**, significant degradation did not occur in any of the sample sets.

## TABLE II

**Alfaxalone Concentrations Measured After Storage of an Alfaxalone/2-Hydroxypropyl-β-cyclodextrin Complex in Either Aqueous Phosphate Buffer or Water at 4° (24 hrs), 25° (24 hrs) and 123° (autoclaved for 35 minutes, 17 psi). The Initial Concentration of Alfaxalone in these Samples was 20 mg/mL.***

| Treatment | Solvent | Amount Remaining |
|---|---|---|
| 4° | $H_2O$ | 102.5% |
| 25° | $H_2O$ | 98.0% |
| 123° | $H_2O$ | 100.5% |
| 4° | Phosphate Buffer | 98.0% |
| 25° | Phosphate Buffer | 98.0% |
| 123° | Phosphate Buffer | 97.0% |

***Calculated from the mean of four determinations.**

To ensure that conditions used in the autoclaving were bacteriocidal, solutions of HPCD (40% w/v) were inoculated with Staphylococcus epidermidus and Escherichia coli, both at levels greater than $10^7$ organism/mL. These solutions were then sealed in ampoules and subjected to steam sterilization using the same conditions as described above. No bacterial growth was detected after incubating autoclaved samples on blood agar plates overnight.

These results indicate that the useful anesthetic agent alfaxalone can be conveniently prepared in aqueous solution of selected cyclodextrins in accord with the present invention. This process eliminates the need to include Cremophor™ in the parenteral dosage formulations. Importantly, these results offer the potential advantage of new formulations which manifest fewer toxic reactions, as borne out by animal tests detailed hereinbelow. In addition, the degree to which alfaxalone is solubilized by selected cyclodextrins in accord with this invention is much greater than that achieved by the Cremophor formulation and there is no need to include the less potent alfadolone acetate as a solubilizing agent.

The present studies also indicate that HPCD solubilizes alfaxalone via 1:1 complexation as indicated by the linear phase solubility retention and that liquid formulations of alfaxalone are stable to steam sterilization.

**FURTHER SOLUBILITY STUDIES**

Phase-solubility experiments were conducted by adding excess amounts of alfaxalone to phosphate buffer solutions containing 20% w/v of selected cyclodextrins and sonicating the mixtures for at least one hour. (The phosphate buffer solution was prepared by dissolving 1.183 g $KH_2PO_4$ and 4.320 g $Na_2HPO_4$ in water and diluting to 1 liter.) The mixtures were filtered through .45 μm Millipore syringe filters and diluted and the drug concentrations were measured by reversed phase HPLC methods, as described above.

HPLC Methods for Alfaxalone

| | |
|---|---|
| Wavelength: | 294 nm |
| Mobile phase: | methanol:water (65:35) |
| Flow rate: | 1.5 mL/min |
| Retention time: | ~14 min. |

The results of these studies are summarized in **TABLE III** below.

**TABLE III**

Solubilization of Alfaxalone by 20% w/v Solutions of Selected Cyclodextrins at Room Temperature

| Cyclodextrin | Solubility (mg/mL) | |
|---|---|---|
| maltosyl-β-cyclodextrin mixture | 20.02 | |
| hydroxypropyl-γ-cyclodextrin | 18.41 | 16.76 |
| hydroxypropyl-γ-cyclodextrin | 15.11 | |
| hydroxypropyl-β-cyclodextrin | 22.83 | 23.62 |
| hydroxypropyl-β-cyclodextrin | 24.42 | |

**ALTERNATE METHOD FOR ALFAXALONE INCORPORATION IN HPCD**

Alfaxalone was suspended in a 43.5% w/v solution of hydroxypropyl-β-cyclodextrin which had been adjusted to pH 7 with sodium hydrogen carbonate. The suspension was sonicated for one hour at room temperature and was subsequently filtered. The clear solution was then freeze-dried. The solid material was analyzed by HPLC and was found to contain 116.4 ± 1.1 mg per gram complex. Chromatography was performed using 2 Perkin-Elmer HS-3 C18 pecosphere cartridges (3.3 cm x 4 mm i.d., 3 μm particle size) in series. The mobile phase contained 60:35 methanol:water and the flow rate was 1 mL/min. UV detection at 208 nm was used in the quantitation. Butylparaben served as an internal standard. The retention time for alfaxalone on the above system was 5.9 minutes while that for the internal standard was 3.6 minutes.

**ILLUSTRATIVE PREPARATION OF ALFAXALONE/HPCD COMPLEX FOR INJECTION**

To a vial containing 5.95 g of an alfaxalone/HPCD complex (126 mg alfaxalone/g complex), corresponding

to 750 mg of the active steroid per vial, 12 mL of sterile water for injection is added. This generates an approximately isotonic solution containing 43.5% w/v HPCD and alfaxalone at a concentration of 46.88 mg/mL. The total volume of the solution after dilution is 16.1 mL. The time required to dissolve the complex is variable, but 5 to 15 minutes of agitation is generally used. The solutions produced are relatively viscous (dynamic viscosity is approximately 2.87 mPa sec) but are syringable through any reasonable gauge needle (≦ 21).

## ANESTHETIC TESTING OF ALFAXALONE/HPCD COMPLEX IN THE RAT

The anesthetic effects of alfaxalone as a water soluble formulation with hydroxypropyl-β-cyclodextrin (HPCD) were compared to the commercially available SAFFAN™ preparation using the intravenous and intraperitoneal administration routes in adult male and female Sprague Dawley rats. Parameters monitored included: a) duration of loss of righting response; b) duration of absence of response to abdominal skin pinch; c) duration of absence of response to auditory stimulus; and d) difference in time of tail flick response to 55°C stimulus before drug and 15 minutes alter gaining righting response.

The water soluble formulation was prepared as a 20% w/v solution of HPCD containing 12 mg/mL of alfaxalone (Batch # 062-143-2X, assayed to contain 83 mg alfexalone/g) with deionized, purified water. The SAFFAN™ preparation was administered directly from the commercial vial (Batch 039 Exp. 3/89) and was specified to contain 0.9% w/v alfaxalone and 0.3% w/v alfadolone acetate. Both preparations were administered via tail vein injection (i.v.) or via intraperitoneal (i.p.) injection in doses calculated from individual animal body weight.

**Table IV** summarizes the duration of loss of righting response. There was no consistent difference in tail flick response before and after either drug formulation treatment. This indicates that recovery was rapid and motor response to adverse stimuli appeared normal shortly after gaining righting response.

Response to auditory stimuli was comparable between formulations as was the pinch response. These responses were used to estimate dose required for surgical anesthesia. With the i.v. route, an adequate anesthetic level for incision was only obtained with 1 mL/kg and was sustained for 5-10 minutes but not 15 minutes in all animals. The i.p. route required a larger dose of drug, with 4 mL/kg marginally effective in female rats and the 6 mL/kg dose adequate for surgery in 3 of 4 injected females. The 8 mL/kg dose induced surgical anesthesia in 3 of 4 and 2 of 3 male rats treated with alfaxalone/HPCD or SAFFAN™, respectively.

## TABLE IV

Duration of Loss of Righting Response in Sprague Dawley Rats after Alfaxalone Formulated in 2-Hydroxypropyl-β-Cyclodextrin or as SAFFAN™

| Route/Sex | Dose (mL/kg) | Duration Lost Righting Response (minutes) | |
|---|---|---|---|
| | | SAFFAN (9 mg/mL Alfaxalone + 3 mg/mL Alfadolone Acetate in Cremophor) | Alf-HPCD (12 mg/mL Alfaxalone, 20% HPCD) |
| i.v. female | 0.25 | 6.1 ± 1.3* | 8.3 ± 1.3* |
| | 0.50 | 13.0 ± 1.0 | 14.6 ± 1.4 |
| | 1.00 | 28.0 ± 0.8 | 28.1 ± 2.5 |
| i.v. male | 0.25 | 10.6 ± 2.1 | 10.1 ± 1.0 |
| | 0.50 | 20.7 ± 2.7 | 19.8 ± 4.1 |
| | 1.00 | 33.7 ± 2.0 | 29.0 ± 2.4 |
| i.p. female | 2.0 | 34.1 ± 9.7 | 39.8 ± 2.1 |
| | 4.0 | 122.2 ± 10.8 | 123.5 ± 16.9 |
| | 6.0 | 177.5 ± 5.8 | 235.2 ± 9.3 |
| i.p. male | 2.0 | 7.3 ± 7.5 | Not measurable |
| | 4.0 | 49.3 ± 20.8 | 74.0 ± 20.5 |
| | 8.0 | 132.2 ± 1.5 | 185.8 ± 18.9 |

*mean ± SD

### ADDITIONAL ANESTHETIC TESTING OF ALFAXALONE/HPCD COMPLEX IN THE RAT

Alfaxalone-HPCD was administered as either an i.m. or an i.v. preparation. The i.m. mixture was made as follows: a 25 mg/mL solution was prepared by dissolving 500 mg alfaxalone-HPCD (incorporation rate of 122 mg alfaxalone/g complex) to yield a final HPCD concentration of 20% (w/v). The i.v. mixture was similarly prepared so that the final concentration of alfaxalone-HPCD was 40 mg/mL Adult male Sprague-Dawley rats (n = 2 per group) received i.m. injections at 5, 10 or 20 mg/kg and i.v. doses at either 10 or 20 mg/kg.

Alfaxalone-HPCD was evaluated for its anesthetic properties by observing each animal and noting behavioral changes characteristic of planes of anesthesia throughout the course of drug action. These observations included corneal, tail- and foot-pinch and startle reflexes and abdominal tone. Quantitatively, each animal was evaluated from time of injection to the time at which the animal was able to be reclined onto its back (supination) and from injection until the rat was able to voluntarily return to a prone position (onto both the fore- and hindlegs).

The results for the quantitative portion of the experiment are expressed in the following **Table V.** Each value represents the mean time (in minutes) per occurrence and numbers in parentheses indicate the absolute value for each animal.

## TABLE V

**Alfaxalone-HPCD (i.m.)**

| Dose | Supination | Pronation to Forelegs | Pronation to Hindlegs |
|---|---|---|---|
| 5 mg/kg | 8.8 (5,13) | 16.8 (7,27) | 17.0 (8,27) |
| 10 mg/kg | 10.7 (9,13) | 35.9 (21,51) | 37.8 (25,51) |
| 20 mg/kg | 5.4 (5,6) | 80.0 (75,85) | 85.0 (75,95) |

**Alfaxalone-HPCD (i.v.)**

| Dose | Supination | Pronation to Forelegs | Pronation to Hindlegs |
|---|---|---|---|
| 10 mg/kg | 0.32 (0.3,0.33) | 20.6 (20,21) | 20.6 (20,21) |
| 20 mg/kg | 0.20 (0.2,0.2) | 49.6 (46,54) | 49.6 (46,64) |

Qualitatively, all animals which received an i.m. injection of alfaxalone-HPCD (regardless of the dosage) maintained corneal reflexes, abdominal tone, pinch responses and startle reflexes throughout the time course. Despite the shallow plane of anesthesia, rats were docile and easily handled. Once the animals became ambulatory, there was no carryover of anesthesia (i.e. staggering or momentary laxing of the hindlegs). However, surgery would not be possible at these selected i.m. dosages of alfaxalone-HPCD.

Rats receiving an i.v. dose of alfaxalone-HPCD at 10 mg/kg lost abdominal tone by 1.5 minutes and returned to the normal state by 6 minutes. Corneal reflexes were constantly present and respiration was even and clear of raspiness. Those animals which received alfaxalone-HPCD (i.v.) at 20 mg/kg lost abdominal tone by 30 seconds, which returned at 19.5 minutes. Pinch responses disappeared within 1 minute and returned by 10 minutes.

These preliminary data indicate that (1) alfaxalone can indeed be solubilized in a cyclodextrin moiety, and (2) this alfaxalone-HPCD complex is capable of eliciting anesthesia.

### COMPARISON OF HISTAMINE RELEASE PROVOKED BY SAFFAN™ AND ALFAXALONE/HPCD IN THE DOG

This study was undertaken to compare the SAFFAN™ (Cremophor vehicle) with a representative water-based, non-surfactant composition of the present invention.

Six dogs were divided at random so that two received the SAFFAN™ preparation (9 mg alfaxalone and 3 mg alfadolone per mL, in Cremophor EL solvent), while the remaining four dogs received an alfaxalone/HPCD complex which was dissolved in distilled water so that each mL contained 9 mg alfaxalone (Batch # 062-143-2X). The dose was chosen to be the same as that recommended for the commercial preparation in the cat. (SAFFAN™ is contraindicated in the dog because of allergic response.)

The anesthetics (0.75 mL/kg) were administered at 20 mL/minute through an intravenous catheter. A balanced electrolyte solution was administered to the dogs throughout the experiment at a rate of 10 mL/kg/hour. Continuous ECG tracings were made prior to induction of anesthesia until at least 30 minutes later. Blood pressure was measured as soon after induction of anesthesia as possible using a Doppler flow detector. This method is considered inaccurate at systolic blood pressures below 50 mm Hg. Respiratory rate was recorded at least every five minutes. The time to extubation and return of ambulation was recorded.

Blood samples-were taken from the jugular vein of each dog before placement of intravenous catheters and again 15 minutes after the induction of anesthesia. The blood was collected in EDTA and immediately spun down to allow plasma separation. The plasma was then frozen at -20°C until shipped in dry ice to be assayed for histamine.

The histamine assay was performed using the radioenzymatic assay described by Faraj et al, J. Nuc. Med.

25, 56-63 (1984) and Faraj et al, Allergy 41, 526-531 (1986).

Both dogs given the SAFFAN™ preparation were administered antihistamine (diphenhydramine, 30 mg) at the end of the initial 30 minutes monitoring period.

The data collected during the six anesthetic inductions are presented in **TABLE VI** below.

The symbol IPPV used in Table VI means "internal positive pressure ventilation".

TABLE VI

| ALPHA CODE | A | B | C | D | E | F |
|---|---|---|---|---|---|---|
| WEIGHT (kg) | 29.3 | 31.3 | 26.7 | 32.4 | 20.7 | 29.3 |
| FORMULATION | ALF/HPCD | ALF/HPCD | ALF/HPCD | SAFFAN | SAFFAN | ALF/HPCD |
| DOSE (mL) | 22.0 | 23.5 | 20.0 | 24.3 | 15.5 | 22.0 |
| TIMES [minutes] | | | | | | |
| extubation | 35 | 18 | 18 | 100 | 105 | 37 |
| standing | 42 | 45 | 43 | 165 | 200 | 40 |
| HISTAMINE [ng/mL] | | | | | | |
| pre-administration | 0.092 | 0.080 | 3.640 | 5.600 | 7.410 | 5.530 |
| 15 minutes post-administration | 1.152 | 4.616 | 3.660 | 88.430 | 90.130 | 7.800 |
| HEMATOCRIT [%] | | | | | | |
| pre-administration | 43 | 44 | 49 | 43 | 47 | 40 |
| 15 minutes post-administration | 37 | 37 | 40 | 61 | 55 | 38 |
| 24 hours | 37 | 42 | 27 | 47 | 45 | 49 |

| ALPHA CODE | A | B | C | D | E | F |
|---|---|---|---|---|---|---|
| WEIGHT (kg) | 29.3 | 31.3 | 26.7 | 32.4 | 20.7 | 29.3 |
| FORMULATION | ALF/HPCD | ALF/HPCD | ALF/HPCD | SAFFAN | SAFFAN | ALF/HPCD |
| DOSE (mL) | 22.0 | 23.5 | 20.0 | 24.3 | 15.5 | 22.0 |
| HEART RATE [BPM] | | | | | | |
| pre-administration | 120 | 140 | 100 | 92 | 100 | 156 |
| 1 min | 180 | 140 | 156 | 164 | 164 | 168 |
| 2 min | 162 | 140 | 152 | 186 | 156 | 174 |
| 5 min | 162 | 128 | 156 | 208 | 144 | 135 |
| 10 min | 144 | 108 | 140 | 204 | 164 | 120 |
| 15 min | 132 | 100 | 130 | 208 | 148 | 126 |
| 20 min | | 100 | 138 | 198 | 162 | 128 |
| 25 min | | | | 180 | 136 | 118 |
| 30 min | | | | 167 | 152 | 132 |
| RESPIRATORY RATES [BPM] | | | | | | |
| 1 min | 12 | | 16 | 12 | IPPV | 16 |
| 2 min | | 16 | 8 | IPPV | IPPV | |
| 5 min | 12 | 12 | 12 | IPPV | IPPV | 16 |
| 10 min | 20 | 16 | 36 | IPPV | IPPV | 16 |
| 15 min | 20 | 32 | 64 | IPPV | IPPV | 18 |
| 20 min | | | | IPPV | IPPV | 22 |
| 25 min | | | | IPPV | | 28 |
| 30 min | | | | | | 18 |

| ALPHA CODE | A | B | C | D | E | F |
|---|---|---|---|---|---|---|
| WEIGHT (kg) | 29.3 | 31.3 | 26.7 | 32.4 | 20.7 | 29.3 |
| FORMULATION | ALF/HPCD | ALF/HPCD | ALF/HPCD | SAFFAN | SAFFAN | ALF/HPCD |
| DOSE (mL) | 22.0 | 23.5 | 20.0 | 24.3 | 15.5 | 22.0 |
| BLOOD PRESSURE [MM HG] | | | | | | |
| 1 min | 140 | | 130 | TOO | TOO | |
| 2 min | | 174 | | LOW | LOW | 38 |
| 5 min | 120 | 140 | 108 | TO | TO | 48 |
| 10 min | 120 | 130 | 100 | BE | BE | 64 |
| 15 min | 118 | 140 | 105 | DETECTED | DETECTED | 98 |
| 20 min | | | 115 | * | * | 106 |
| 25 min | | | | * | * | 112 |
| 30 min | 125 | | | * | * | 110 |

The remarkable data are the anesthetic times, blood pressures and the histamine assays when compared between the two preparations. The two dogs [D and E] given the SAFFAN™ product showed very elevated plasma histamine levels, profound hypotension, and prolonged anesthetic times. Only one of the dogs given the alfaxalone/HPCD formulation [F] developed hypotension, and this was of much lower order than that provoked by SAFFAN™. Serum blood chemistry data showed no consistent pattern of change between the pre- and 24-hours-post anesthetic values except a similar decrease in total calcium, which decreased 10-13% in all dogs. None of the experimental dogs had any cardiac dysrhythmias as judged by inspection of the polygraph tracing.

In both of the SAFFAN™ dogs, the skin appeared to become erythematous by about 20 minutes after the induction of anesthesia. This abated after the administration of the antihistamine, diphenhydramine, at 30 minutes post-induction. When recovered, neither dog displayed any pruritus.

The experimental data clearly indicate both dogs given the SAFFAN™ preparation had sufficient histamine release to affect blood pressure and ventilation. Blood pressure was so decreased that the non-invasive method used could not detect any blood flow and so no data was obtained in the first 30 minutes of anesthesia. Ventilation was affected in that, despite attempts to breath, the SAFFAN™ dogs seemed unable to generate an adequate flow of air and became hypoxic. Manual ventilation was performed but the lungs seemed to have low compliance. These effects on ventilation could have been due to histamine-induced bronchospasms. The reaction of the dogs receiving the SAFFAN™ formulation was clearly a classical anaphylactoid reaction. After thirty minutes, the SAFFAN™ dogs were each given a total of 30 mg diphenhydramine to curtail the histamine actions. Adequate spontaneous ventilation was regained shortly after that.

The prolonged sleep time was "real" but could have been exaggerated by the diphenhydramine administered. Both the SAFFAN™ dogs were able to walk when finally aroused and had an uneventful recovery. The four alfaxalone/HPCD dogs all showed paddling and slight phonation in early recovery but this would most likely be eliminated by proper preanesthetic medication.

The histamine released showed dramatic differences between the two preparations. Normal histamine values for dogs are 0.01 - 2.0 ng/mL Four of the experimental dogs had control levels above this [C D E F]. On the basis of one sample, this cannot be explained. Dogs A and B both exhibited elevations of histamine after being anesthetized but these were still only just above the normal levels, especially compared with the concentrations achieved by the two SAFFAN™ dogs. The histamine assays were carried out blinded as to the reason for the assays and which dogs were receiving which treatment.

It can be concluded from this study that the alfaxalone/HPCD formulation releases far less histamine in the dog than does the SAFFAN™ preparation. This lesser tendency to release histamine appears to provide greater cardiopulmonary stability.

The fall in blood pressure in dog F appears to be anomalous. There exists a faint possibility that a stopcock was contaminated by the SAFFAN™ preparation and this minute amount of contamination caused the hypotension, which was less than occurred in the SAFFAN™ dogs, but still an undesirable event. Alternatively, the fall in blood pressure could have been caused by the rapid rate of administration. That all dogs were apneic immediately after induction was an indicator of this.

## ALFAXALONE/HPCD IN THE HORSE

The commercial preparation of alfaxalone/alfadolone acetate/Cremophor EL (ALTHESIN™/SAFFAN™) was tested in the horse in 1974 [Eales et al, Proc. Assoc. Vet. Anaesth. GB&I, No. 5, 1-5, (1974)]. Those researchers found that there was considerable excitement in recovery, with special emphasis on auditory sensitivity. The present study sought to determine whether use of the alfaxalone/HPCD formulation would give better results.

In the first stage, two horses were anesthetized after heavy premedication with xylazine (0.6 mg/kg). The induction dose of alfaxalone used was 1.5 mg/kg infused over 20 seconds. The solution administered was prepared from alfaxalone/HPCD complex Batch No. 096-78-7 containing 100 mg alfaxalone/g complex, by dilution of the powder with distilled water (100 mL water added to 30 g complex). The administered complex thus contained 3% alfaxalone and 27% HPCD. The horses were then intubated and maintained with halothane for an hour to permit recovery to be free of any alfaxalone residual effects. Heart rate, respiratory rate, blood pressure, ECG and ventilation were monitored throughout the anesthesia period.

In the second stage, the recovery characteristics of a single bolus of alfaxalone in two horses was monitored under similar premedication. The same parameters as in the first stage were monitored.

In the third stage, the same premedication and induction dose of alfaxalone was administered and then the administration of alfaxalone was repeated at one-half to one-third of the induction dose. Cardiopulmonary parameters were monitored as before.

To provide some comparison in the above stages, the two horses were anesthetized with thiamylal sodium (8 mg/kg) after the same premedication as before and a similar monitoring regime followed.

A third horse was obtained and the same regimen was followed as in the third stage, but three "top-ups" of one-third induction dose were administered at 10 minute intervals. Similar monitoring as above was continued.

Blood pressure was measured from an acutely placed antraarterial catheter and read through a transducer/digital readout system which included an ECG tracing oscilloscope.

The xylazine (0.6 mg/kg) i.v. produced good sedation without ataxia in all animals. No heart blocks were seen.

Induction in all horses was similar, with extensor rigidity similar to that seen in xylazine-ketamine anesthesia being observed. However, in the alfaxalone inductions, the horses quickly relaxed. The induction characteristics were rated excellent.

With the exception of one horse, all maintenance periods were uneventful. One of the horses receiving a "top-up" dose had prolonged ventricular ectopic beats and bigeminy. The horse was breathing air and in lateral recumbency; it appeared "dusky" and an arterial blood gas showed a $P_aO_2$ of 27 mm Hg. Oxygen was administered by nasal insufflation and the arrhythmia resolved. This was the only ECG irregularity observed in the study and was ascribed to hypoxia. All later data was collected with the horses receiving oxygen supplementation to avoid this sort of occurrence.

Ventilation of the horses throughout the anesthesia period was slow, i.e. usually one very large breath per minute. All $P_aCO_2$ values were in the low 50's indicating ventilation was adequate despite the low ventilatory rate.

A very interesting phenomena was the hypertension seen in all alfaxalone studies. Because of this phenomena, the thiamylal study was undertaken to determine if the same effect would be observed with use of a barbiturate anesthetic. The thiamylal study did in fact show the same effect. This could be due to the xylazine premedication. However, in the "top-up" studies it should be noted that the blood pressure rises immediately after each bolus of alfaxalone. It did not present a problem in any horse studied.

Several horses showed sudden tonic movement at the end of the anesethetic period but quickly relaxed. No effort was made in these studies to evaluate the level of the anesthesia, which appeared to be light throughout.

Four of the five alfaxalone alone recoveries would be classified as excellent. The horses came to sternal recumbency, remained there for some time appearing fully alert, then stood in a smooth manner. The halothane associated recoveries were good but typical of an inhalant recovery. One horse had an average recovery with six attempts to stand before being successful. This was the same episode where the horse was severely hypoxic, which may have been a contributing factor.

In all of the recoveries, there was no suggestion of an auditory sensitivity. Banging on the recovery box door would stimulate the horses, but no more than after an inhalant anesthetic. It seems from the study that topping-up with alfaxalone would probably not increase recovery time or detract from the quality of the recovery.

Overall, the alfaxalone/HPCD formulation gave impressive results. Induction was excellent and no auditory sensitivity was noted.

In some species, for example in the horse and in the cat, administration of steroidal anesthetic formulations such as SAFFAN™ has provoked hyperexcitement or delirium in addition to the various manifestations of an "allergic response" as discussed hereinabove. It is not clear whether the hyperexcitement/delirium reaction should, strictly speaking, be classified as an "allergic response". Nevertheless, such an untoward reaction, like the auditory sensitivity noted in the horse, is clearly associated with surfactant-containing steroidal anesthetic formulations such as SAFFAN™. The use of steroidal anesthetic/hypnotic complexes with selected cyclodextrins in accord with the present invention prevents or mitigates such untoward reactions as well as the more classic manifestations of an allergic or hypersensitivity reaction.

The anesthetic/hypnotic compositions of the present invention comprise an anesthetically/hypnotically effective amount of at least one steroidal anesthetic/hypnotic complexed with cyclodextrin selected from the group consisting of hydroxypropyl, hydroxyethyl, glucosyl, maltosyl and maltotriosyl derivatives of β- and γ-cyclodextrin, the amount of the steroidal anesthetic/hypnotic being from about 0.5% to about 15% by weight of the amount of the cyclodextrin. In preferred complexes, the steroid is from about 5 to about 15% by weight of the amount of the cyclodextrin. The compositions, which are substantially free of surface active agents, are hypoallergenic. Various non-surfactant carrier materials may, however, be present in the instant pharmaceutical or veterinary formulations, so long as the carriers are non-toxic and do not act as allergens in the compositions. Selection of an appropriate carrier or carriers will be readily apparent to those of ordinary skill in the art and will obviously depend upon the selected route of administration. Thus, for example, tablets or lozenges for buccal or sublingual administration, suppositories for vaginal or rectal administration, gels, ointments or solutions for nasal or ophthalmic administration, and solutions for various injectable routes of administration may be formulated using carriers appropriate for the desired route.

Injectable compositions are preferred, particularly those formulated for intravenous, intramuscular or subcutaneous administration. The injectable formulations are sterile and pyrogen-free, and contain water as the primary carrier material. These aqueous injection solutions contain from about 10% to about 60%, preferably from about 20% to about 50%, most preferably from about 20% to about 30%, w/v or w/w cyclodextrin and are prepared in accord with accepted pharmaceutical procedures, for example as described in Remington's Pharmaceutical Sciences, seventeenth edition, ed. Alfonso R. Gennaro, Mack Publishing Company, Easton, PA, pp. 1518-1552(1985). Concentrations of cyclodextrin above about 60% are generally too viscous for injection with a reasonable gauge needle. The aqueous sterile injection solutions may further contain anti-oxidants, buffers, bacteriostats, isotonicity adjusters and like additions acceptable for parenteral formulations. The general requirements that the compositions be substantially free of surfactants and that the carriers be non-toxic and hypoallergenic of course apply. Typical injectable formulations contain the selected steroid/cyclodextrin complex(es), water and a preservative.

Various unit dose and multidose containers, e.g. sealed ampoules and vials, may be used, as is well-known in the art. The essential ingredients of the sterile injectable formulation, i.e. the steroidal anesthetic/hypnotic, water and selected cyclodextrin, may be presented in a variety of ways, just so long as the solution ultimately administered to the vertebrate animal contains the appropriate amounts of the essential ingredients. Thus, for example, the steroid/cyclodextrin/water formulation may be presented in a unit dose or multidose container, ready for injection. As another example, a concentrated solution of steroid/cyclodextrin/water may be presented in a separate container from a diluting liquid (water or cyclodextrin/water) designed so that the contents can be combined to give a formulation containing appropriate amounts for injection. As another alternative, the steroid/cyclodextrin complex may be provided in a freeze-dried condition in one container, while a separate container contains diluting liquid (water or cyclodextrin/water, depending on the amount of cyclodextrin in the other container), again designed so that the contents can be combined to give a formulation containing the appropriate amounts of the essential ingredients. In any event, the contents of each container will be sterile.

Generally speaking, the therapeutic dosage ranges for administration of steroidal anesthetics/hypnotics in the injectable formulations described herein will be less than those characteristically used for administration of the steroids in surfactant vehicle. Naturally, such therapeutic dosage ranges will vary with the size and species of the animal, the condition for which the formulation is administered, the type of injectable administration employed and the like. The quantity of given dosage form needed to deliver the desired dose of active ingredients will of course depend upon the concentration of the drug in the formulation. Whatever the formulation employed, appropriate dosage levels can be determined by routine experimentation.

By way of example, when the steroid selected for use herein is alfaxalone, intramuscular injection may be conveniently used to elicit a state of sedation and hypnosis (e.g. as a preanesthetic), while intravenous administration may be advantageously employed to elicit surgical anesthesia. Further doses may be administered intravenously or intramuscularly to extend the anesthetic/hypnotic effect. Continuous dosing may also be utilized, typically by i.v. drip. Illustrative dosage levels for initial administration may be calculated from those known from the literature for alfaxalone in the ALTHESIN™/SAFFAN™ formulation. Of the 12 mg/mL total steroid content of SAFFAN™, there are 9 mg/mL of alfaxalone and 3 mg/mL of alfadolone acetate (which has one-third to one-half the anesthetic activity of alfaxalone). Thus, the total anesthetic effect of the commercial formulation is equivalent to that of about 10 or 10.5 mg/mL alfaxalone. Such actual concentrations of alfaxalone are readily achieveable in the present surfactant-free formulations, but could not be obtained previously in the absence of surfactants. Thus, the actual alfaxalone content of the instant formulations may be from about 12% to about 17% less than the total steroid dose previously administered in SAFFAN™, to achieve an equivalent effect. Amounts of alfaxalone in the instant formulations equal to the total steroid content previously administered would be expected to produce a deeper or more prolonged hypnosis/anesthesia. As would be expected, relatively higher doses are required for intramuscular or subcutaneous injection than are needed by the intravenous route.

While the invention has been described in terms of various preferred embodiments, the skilled artisan will appreciate that various modifications, substitutions, omissions and changes may be made. Accordingly, it is intended that the scope of the present invention be limited solely by the scope of the following claims.

## Claims

### Claims for the following Contracting States : AT, BE, CH, DE, DK, FR, GB, IT, LI, LU, NL, SE

1. A parenterally acceptable hypoallergenic steroidal anesthetic or hypnotic composition for use in eliciting anesthesia or a hypnotic response in a vertebrate animal without provoking a significant allergic response, which composition comprises an anesthetically or hypnotically effective amount of a steroidal anesthetic or hypnotic complexed with cyclodextrin selected from the group consisting of hydroxypropyl, hydroxyethyl, glucosyl, maltosyl and maltotriosyl derivatives of β- and γ-cyclodextrin, the amount of said steroidal anesthetic or hypnotic being from 0.5% to 15% by weight of the amount of said cyclodextrin, said composition being substantially free of surface active agents.

2. A composition according to Claim 1, formulated as an injectable aqueous solution having a total cyclodextrin content of from 10% to 60% by weight.

3. A composition according to Claim 2, wherein the total cyclodextrin content of the solution is from 20% to 50% by weight.

4. A composition according to Claim 3, wherein the total cyclodextrin content of the solution is from 20% to 30% by weight.

5. A composition according to any one of the preceding claims, wherein the steroidal anesthetic or hypnotic comprises alfaxalone.

6. A composition according to Claim 5, wherein the amount of alfaxalone is from 5% to 15% by weight the amount of cyclodextrin.

7. A composition according to any one of the preceding claims, wherein the cyclodextrin comprises hydroxypropyl-β-cyclodextrin.

8. A composition according to any one of Claims 1 to 6, wherein the cyclodextrin comprises hydroxypropyl-γ-cyclodextrin.

9. A composition according to any one of Claims 1 to 6, wherein the cyclodextrin comprises maltosyl-β-cyclodextrin.

10. A composition according to any one of Claims 1 to 6, wherein the cyclodextrin comprises a mixture of maltosyl-β-cyclodextrin and dimaltosyl-β-cyclodextrin.

11. A composition according to any one of the preceding claims, adapted for intravenous intramuscular or subcutaneous administration.

12. A method for preparing a parenterally acceptable hypoallergenic steroidal anesthetic or hypnotic composition, said method comprising complexing an anesthetically or hypnotically effective amount of a steroidal anesthetic or hypnotic with cyclodextrin selected from the group consisting of hydroxypropyl, hydroxyethyl, glucosyl, maltosyl and maltotriosyl derivatives of β- and γ-cyclodextrin, followed by formulating the resultant complex into a parenterally acceptable composition wherein the amount of said steroidal anesthetic or hypnotic is from 0.5% to 15% by weight of the amount of said cyclodextrin in said composition, said composition being substantially free of surface active agents.

13. A method according to Claim 12, said parenterally acceptable hypoallergenic composition being formulated as an injectable aqueous solution having a total cyclodextrin content of from 10% to 60% by weight.

14. A method according to Claim 13, wherein the total cyclodextrin content of the solution is from 20% to 50% by weight.

15. A method according to Claim 14, wherein the total cyclodextrin content of the solution is from 20% to 30% by weight.

16. A method according to any one of Claims 12 to 15, wherein said composition is formulated for intravenous, intramuscular or subcutaneous administration.

**Claims for the following Contracting States : ES, GR**

1. A method for preparing a parenterally acceptable hypoallergenic steroidal anesthetic or hypnotic composition, said method comprising complexing an anesthetically or hypnotically effective amount of a steroidal anesthetic or hypnotic with cyclodextrin selected from the group consisting of hydroxypropyl, hydroxyethyl, glucosyl, maltosyl and maltotriosyl derivatives of β- and γ-cyclodextrin, followed by formulating the resultant complex into a parenterally acceptable composition wherein the amount of said steroidal anesthetic or hypnotic is from 0.5% to 15% by weight of the amount of said cyclodextrin in said composition, said composition being substantially free of surface active agents.

2. A method according to Claim 1, wherein the parenterally acceptable hypoallergenic composition is formulated as an injectable aqueous solution having a total cyclodextrin content of from 10% to 60% by weight.

3. A method according to Claim 2, wherein the total cyclodextrin content of the solution is from 20% to 50% by weight.

4. A method according to Claim 3, wherein the total cyclodextrin content of the solution is from 20% to 30% by weight.

5. A method according to any one of Claims 1 to 4, wherein said composition is formulated for intravenous, intramuscular or subcutaneous administration.

6. A method according to any one of the preceding claims, wherein the steroidal anesthetic or hypnotic comprises alfaxalone.

7. A method according to Claim 6, wherein the amount of alfaxalone is from 5% to 15% by weight the amount of cyclodextrin.

8. A method according to any one of the preceding claims, wherein the cyclodextrin comprises hydroxypropyl-β-cyclodextrin.

9. A method according to any one of Claims 1 to 7, wherein the cyclodextrin comprises hydroxypropyl-γ-cyclodextrin.

10. A method according to any one of Claims 1 to 7, wherein the cyclodextrin comprises maltosyl-β-cyclodextrin.

11. A method according to any one of Claims 1 to 7, wherein the cyclodextrin comprises a mixture of maltosyl-β-cyclodextrin and dimaltosyl-β-cyclodextrin.

## Patentansprüche

### Patentansprüche für folgende Vertragsstaaten : AT, BE, CH, DE, DK, FR, GB, IT, LI LU, NL, SE

1. Parenteral akzeptable, hypoallergene steroide anästhetische oder hypnotische Zusammensetzung zur Verwendung beim Hervorrufen einer Anästhesie oder einer hypnotischen Reaktion in einem Vertebraten, ohne dabei eine signifikante allergische Reaktion auszulösen, welche Zusammensetzung eine anästhetisch oder hypnotisch wirksame Menge eines steroiden Anästhetikums oder Hypnotikums enthält, die mit Cyclodextrin komplexiert sind, welches aus der Gruppe gewählt ist, die aus Hydroxypropyl-, Hydroxyethyl-, Glucosyl-, Maltosyl- und Maltotriosyl-Derivaten von β- und γ-Cyclodextrin besteht, welche Menge des steroiden Anästhetikums oder Hypnotikums von 0,5 % bis 15 Gew.-% der Menge des Cyclodextrins ist, welche Zusammensetzung von oberflächenaktiven Mitteln im wesentlichen frei ist.

2. Zusammensetzung nach Anspruch 1, formuliert als eine injizierbare wasserige Lösung, die einen Gesamtcyclodextringehalt von 10 % bis 60 Gew.-% besitzt.

3. Zusammensetzung nach Anspruch 2, in der der Gesamtcyclodextringehalt der Lösung von 20 % bis 50 Gew.-% ist.

4. Zusammensetzung nach Anspruch 3, in der der Gesamtcyclodextringehalt der Lösung von 20 % bis 30 Gew.-% ist.

5. Zusammensetzung nach irgendeinem der vorhergehenden Ansprüche, in der das steroide Anästhetikum oder Hypnotikum Alfaxalon umfaßt.

6. Zusammensetzung nach Anspruch 5, in der die Menge an Alfaxalon von 5 % bis 15 Gew.-% der Cyclodextrinmenge ist.

7. Zusammensetzung nach irgendeinem der vorhergehenden Ansprüche, in der das Cyclodextrin Hydroxypropyl-β-cyclodextrin umfaßt.

8. Zusammensetzung nach irgendeinem der Ansprüche 1 bis 6, in der das Cyclodextrin Hydroxypropyl-γ-cyclodextrin umfaßt.

9. Zusammensetzung nach irgendeinem der Ansprüche 1 bis 6, in der das Cyclodextrin Maltosyl-β-cyclodextrin umfaßt.

10. Zusammensetzung nach irgendeinem der Ansprüche 1 bis 6, in der das Cyclodextrin ein Gemisch aus Maltosyl-β-cyclodextrin und Dimaltosyl-β-cyclodextrin umfaßt.

11. Zusammensetzung nach irgendeinem der vorhergehenden Ansprüche, adaptiert für intravenöse, intramuskuläre oder subcutane Verabreichung.

12. Verfahren zum Herstellen einer parenteral akzeptablen, hypoallergenen steroiden anästhetischen oder hypnotischen Zusammensetzung, welches Verfahren umfaßt: Komplexieren einer anästhetisch oder hypnotisch wirksamen Menge eines steroiden Anästhetikums oder Hypnotikums mit Cyclodextrin, welches von der Gruppe ausgewählt ist, die aus Hydroxypropyl-, Hydroxyethyl-, Glucosyl-, Maltosyl-und Maltotriosyl-Derivaten von β- und γ-Cyclodextrin besteht, gefolgt von Formulieren des erhaltenen Komplexes zu einer parenteral akzeptablen Zusammensetzung, in der die Menge des steroiden Anästhetikums oder Hypnotikums von 0,5 % bis 15 Gew.-% der Menge des Cyclodextrins in der Zusammensetzung ist, welche Zusammensetzung von oberflächenaktiven Mitteln im wesentlichen frei ist.

13. Verfahren nach Anspruch 12, wobei die parenteral akzeptable, hypoallergene Zusammensetzung als eine injizierbare wässerige Lösung formuliert wird, die einen Gesamtcyclodextringehalt von 10 % bis 60 Gew.-% besitzt.

14. Verfahren nach Anspruch 13, bei dem der Gesamtcyclodextringehalt der Lösung von 20 % bis 50 Gew.-% ist.

15. Verfahren nach Anspruch 14, bei dem der Gesamtcyclodextringehalt der Lösung von 20 % bis 30 Gew.-% ist.

16. Verfahren nach irgendeinem der Ansprüche 12 bis 15, bei dem die Zusammensetzung für intravenöse, intramuskuläre oder subcutane Verabreichung formuliert wird.

**Patentansprüche für folgende Vertragsstaaten : ES, GR**

1. Verfahren zum Herstellen einer parenteral akzeptablen, hypoallergenen steroiden anästhetischen oder hypnotischen Zusammensetzung, welches Verfahren umfaßt: Komplexieren einer anästhetisch oder hypnotisch wirksamen Menge eines steroiden Anästhetikums oder Hypnotikums mit Cyclodextrin, welches von der Gruppe ausgewählt ist, die aus Hydroxypropyl-, Hydroxyethyl-, Glucosyl-, Maltosyl-und Maltotriosyl-Derivaten von β- und γ-Cyclodextrin besteht, gefolgt von Formulieren des erhaltenen Komplexes zu einer parenteral akzeptablen Zusammensetzung, in der die Menge des steroiden Anästhetikums oder Hypnotikums von 0,5 % bis 15 Gew.-% der Menge des Cyclodextrins in der Zusammensetzung ist, welche Zusammensetzung von oberflächenaktiven Mitteln im wesentlichen frei ist.

2. Verfahren nach Anspruch 1, wobei die parenteral akzeptable, hypoallergene Zusammensetzung als eine injizierbare wässerige Lösung formuliert wird, die einen Gesamtcyclodextringehalt von 10 % bis 60 Gew.-% besitzt.

3. Verfahren nach Anspruch 2, bei dem der Gesamtcyclodextringehalt der Lösung von 20 % bis 50 Gew.-% ist.

4. Verfahren nach Anspruch 3, bei dem der Gesamtcyclodextringehalt der Lösung von 20 % bis 30 Gew.-% ist.

5. Verfahren nach irgendeinem der Ansprüche 1 bis 4, bei dem die Zusammensetzung für intravenöse, intramuskuläre oder subcutane Verabreichung formuliert wird.

6. Verfahren nach irgendeinem der vorhergehenden Ansprüche, bei dem das steroide Anästhetikum oder Hypnotikum Alfaxalon enthält.

7. Verfahren nach Anspruch 6, bei dem die Menge an Alfaxalon von 5 % bis 15 Gew.-% der Cyclodextrinmenge ist.

8. Verfahren nach irgendeinem der vorhergehenden Ansprüche, bei dem das Cyclodextrin Hydroxypropyl-β-cyclodextrin umfaßt.

9. Verfahren nach irgendeinem der Ansprüche 1 bis 7, bei dem das Cyclodextrin Hydroxypropyl-γ-cyclodextrin umfaßt.

10. Verfahren nach irgendeinem der Ansprüche 1 bis 7, bei dem das Cyclodextrin Maltosyl-β-cyclodextrin umfaßt.

11. Verfahren nach irgendeinem der Ansprüche 1 bis 7, bei dem das Cyclodextrin ein Gemisch aus Maltosyl-β-cyclodextrin und Dimaltosyl-β-cyclodextrin umfaßt.

## Revendications

**Revendications pour les Etats contractants suivants : AT, BE, CH, DE, DK, FR, GB, IT, LI, LU, NL, SE**

1. Composition anesthésiante ou hypnotique stéroïdienne hypoallergénique acceptable par voie parentérale destinée à être utilisée pour engendrer une anesthésie ou une réponse hypnotique chez un animal vertébré sans provoquer une réponse allergique notable, composition qui comprend une quantité à effet anesthésiant ou hypnotique d'un anesthésique ou hypnotique stéroïdien complexé avec une cyclodextrine choisie dans le groupe consistant en dérivés hydroxypropyliques, hydroxyéthyliques, glucosyliques, maltosyliques et maltotriosyliques de β- et γ-cyclodextrines, la quantité dudit anesthésique ou hypnotique stéroïdien étant comprise dans l'intervalle de 0,5 % à 15 % en poids de la quantité de ladite cyclodextrine, ladite composition étant pratiquement dépourvue d'agents tensio-actifs.

2. Composition suivant la revendication 1, formulée en une solution aqueuse injectable ayant une teneur totale en cyclodextrine de 10 % à 60 % en poids.

3. Composition suivant la revendication 2, dans laquelle la teneur totale en cyclodextrine de la solution est comprise dans l'intervalle de 20 % à 50 % en poids.

4. Composition suivant la revendication 3, dans laquelle la teneur totale en cyclodextrine de la solution est comprise dans l'intervalle de 20 % à 30 % en poids.

5. Composition suivant l'une quelconque des revendications précédentes, dans laquelle l'anesthésique ou l'hypnotique stéroïdien comprend de l'alfaxalone.

6. Composition suivant la revendication 5, dans laquelle la quantité d'alfaxalone est comprise dans l'intervalle de 5 % à 15 % en poids de la quantité de cyclodextrine.

7. Composition suivant l'une quelconque des revendications précédentes, dans laquelle la cyclodextrine comprend de l'hydroxypropyl-β-cyclodextrine.

8. Composition suivant l'une quelconque des revendications 1 à 6, dans laquelle la cyclodextrine comprend de l'hydroxypropyl-γ-cyclodextrine.

9. Composition suivant l'une quelconque des revendications 1 à 6, dans laquelle la cyclodextrine comprend de la maltosyl-β-cyclodextrine.

10. Composition suivant l'une quelconque des revendications 1 à 6, dans laquelle la cyclodextrine comprend un mélange de maltosyl-β-cyclodextrine et de dimaltosyl-β-cyclodextrine.

11. Composition suivant l'une quelconque des revendications précédentes, apte à une administration intraveineuse, intramusculaire ou sous-cutanée.

12. Procédé de préparation d'une composition anesthésiante ou hypnotique stéroïdienne hypoallergénique acceptable par voie parentérale, ledit procédé comprenant la complexation d'une quantité à effet anesthésiant ou hypnotique d'un anesthésique ou hypnotique stéroïdien avec une cyclodextrine choisie dans le groupe consistant en dérivés hydroxypropyliques, hydroxyéthyliques, glucosyliques, maltosyliques et maltotriosyliques de β- et γ-cyclodextrines, puis la formulation du complexe résultant en une composition acceptable par voie parentérale dans laquelle la quantité dudit anesthésique ou hypnotique stéroïdien est comprise dans l'intervalle de 0,5 % à 15 % en poids de la quantité de ladite cyclodextrine dans ladite composition, ladite composition étant pratiquement dépourvue d'agents tensio-actifs.

13. Procédé suivant la revendication 12, dans lequel la composition hypoallergénique acceptable par voie parentérale est formulée en une solution aqueuse injectable ayant une teneur totale en cyclodextrine de 10 % à 60 % en poids.

14. Procédé suivant la revendication 13, dans lequel la teneur totale en cyclodextrine de la solution est comprise dans l'intervalle de 20 % à 50 % en poids.

15. Procédé suivant la revendication 14, dans lequel la teneur totale en cyclodextrine de la solution est comprise dans l'intervalle de 20 % à 30 % en poids.

16. Procédé suivant l'une quelconque des revendications 12 à 15, dans lequel la composition est formulée pour l'administration par voie intraveineuse, intramusculaire ou sous-cutanée.

**Revendications pour les Etats contractants suivants : ES, GR**

1. Procédé de préparation d'une composition anesthésiante ou hypnotique stéroïdienne hypoallergénique acceptable par voie parentérale, ledit procédé comprenant la complexation d'une quantité à effet anesthésiant ou hypnotique d'un anesthésique ou hypnotique stéroïdien avec une cyclodextrine choisie dans le groupe consistant en dérivés hydroxypropyliques, hydroxyéthyliques, glucosyliques, maltosyliques et maltotriosyliques de β- et γ-cyclodextrines, puis la formulation du complexe résultant en une composition acceptable par voie parentérale dans laquelle la quantité dudit anesthésique ou hypnotique stéroïdien est comprise dans l'intervalle de 0,5 % à 15 % en poids de la quantité de ladite cyclodextrine, dans ladite composition, ladite composition étant pratiquement dépourvue d'agents tensio-actifs.

2. Procédé suivant la revendication 1, dans lequel la composition hypoallergénique acceptable par voie parentérale est formulée en une solution aqueuse injectable ayant une teneur totale en cyclodextrine de 10 % à 60 % en poids.

3. Procédé suivant la revendication 2, dans lequel la teneur totale en cyclodextrine de la solution est comprise dans l'intervalle de 20 % à 50 % en poids.

4. Procédé suivant la revendication 3, dans lequel la teneur totale en cyclodextrine de la solution est comprise dans l'intervalle de 20 % à 30 % en poids.

5. Procédé suivant l'une quelconque des revendications 1 à 4, dans lequel la composition est formulée pour une administration par voie intraveineuse, intramusculaire ou sous-cutanée.

6. Procédé suivant l'une quelconque des revendications précédentes, dans lequel l'anesthésique ou l'hypnotique stéroïdien comprend de l'alfaxalone.

7. Procédé suivant la revendication 6, dans laquelle la quantité d'alfaxalone est comprise dans l'intervalle de 5 % à 15 % en poids de la quantité de cyclodextrine.

8. Procédé suivant l'une quelconque des revendications précédentes, dans lequel la cyclodextrine comprend de l'hydroxypropyl-β-cyclodextrine.

9. Procédé suivant l'une quelconque des revendications 1 à 7, dans lequel la cyclodextrine comprend de l'hydroxypropyl-γ-cyclodextrine.

10. Procédé suivant l'une quelconque des revendications 1 à 7, dans lequel la cyclodextrine comprend de la maltosyl-β-cyclodextrine.

**11.** Procédé suivant l'une quelconque des revendications 1 à 7, dans lequel la cyclodextrine comprend un mélange de maltosyl-β-cyclodextrine et de dimaltosyl-β-cyclodextrine.

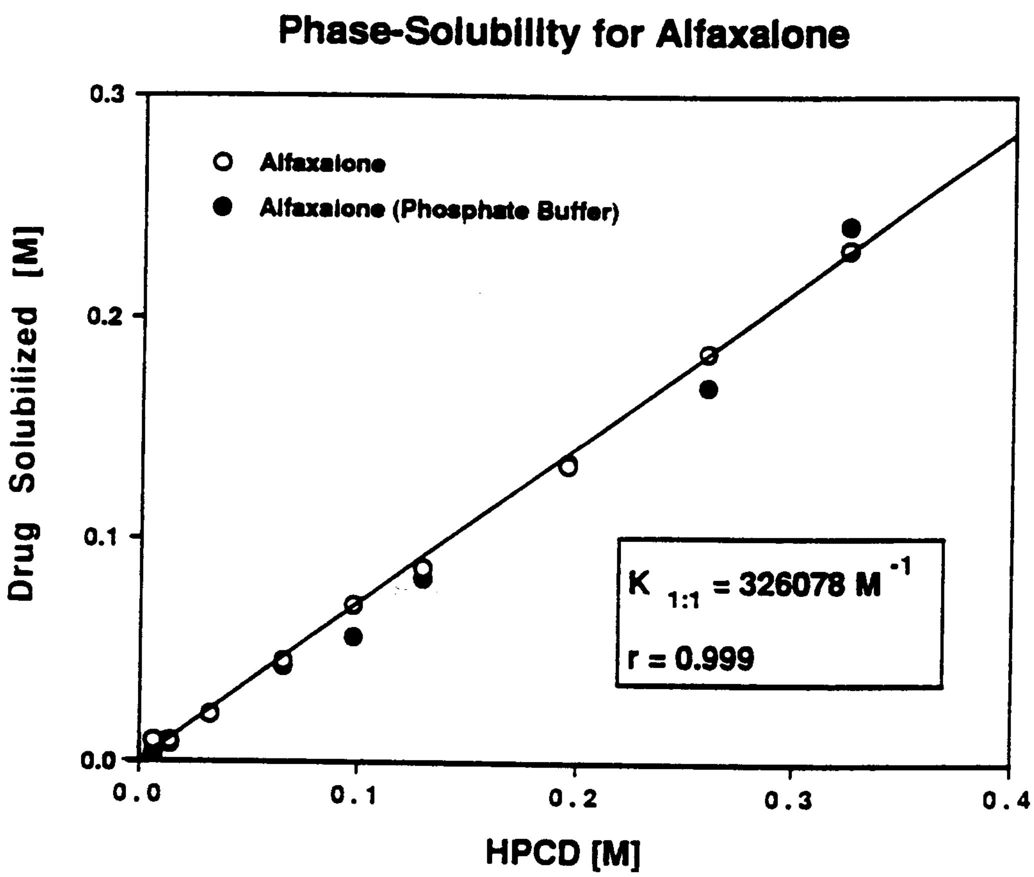
Phase-Solubility for Alfaxalone
Alfaxalone
Alfaxalone (Phosphate Buffer)
K 1:1 = 326078 M -1
r = 0.999
Drug Solubilized [M]
0.0
0.1
0.2
0.3
0.0
0.1
0.2
0.3
0.4
HPCD [M]